# EUROPEAN PATENT APPLICATION

(11) **EP 2 565 277 A1**
(43) Date of publication of application: **06.03.2013**
(21) Application number: 11382284.5
(22) Date of filing: 05.09.2011
(51) Int. Cl.: C12Q 1/68

(54) **Method for predicting radiographic severity in ankylosing spondylitis**

(71) Applicant: Progenika Biopharma, S.A., 48160 Derio (Bizkaia) (ES)
(72) Inventor: Bartolomé Escobar, Nerea, E-48160 Derio (Bizkaia) (ES); Artieda Oseñalde, Marta, E-48160 Derio (Bizkaia) (ES); Szczypiorska, Magdalena, E-48160 Derio (Bizkaia) (ES); Tejedor Hernández, Diego, E-48160 Derio (Bizkaia) (ES); Martínez Martínez, Antonio, E-48160 Derio (Bizkaia) (ES); Simón Buela, Laureano, E-48160 Derio (Bizkaia) (ES)
(74) Representative: ABG Patentes, S.L.

(57) **Abstract**

The method for predicting the risk of an ankylosing spondylitis (AS) patient to develop radiographic severity of AS, radiographic progression of AS, a condition that is associated with the radiographic severity of AS, and/or a condition that is associated with the radiographic progression of AS, is based on the detection of at least one single nucleotide polymorphism (SNP) associated with the evolution of AS. The invention can be used in the design of individualized therapy of AS patients.

## Description

### FIELD OF THE INVENTION

The present invention relates to the field of personalized medicine and provides means for predicting the evolution of ankylosing spondylitis (AS) in a patient. More precisely, the invention provides means and methods for predicting the risk of an AS patient to, and for classifying an AS patient as predisposed or not predisposed to, develop radiographic severity, radiographic progression, a condition that is associated with radiographic severity, and/or a condition that is associated with the radiographic progression of AS, based on the detection of at least one single nucleotide polymorphism (SNP) associated with the evolution of AS.

### BACKGROUND OF THE INVENTION

Ankylosing spondylitis (AS) is a chronic inflammatory rheumatic disease characterized by sacroiliitis, ankylosis of the spine and enthesitis. Radiographic damage in AS may lead to fusion of the spine and to the subsequent impairment of mobility, which considerably reduces the patient's quality of life. While some AS patients remain without significant radiographic changes for many years, others become impaired within few years since disease onset. A number of conditions are associated with radiographic severity and/or progression of AS, such as cervicalgy, enthesitis, dactylitis, tarsitis, sacroiliac syndrome, coxitis, low back pain, upper limbs arthritis, lower limbs arthritis, uveitis, psoriasis, urethritis and inflammatory bowel disease.

Although it is well-established that susceptibility to AS is largely genetically determined (Brown et al., 1997, Arthritis Rheum; 40:1823-28) and the effort made during the last years has led to the discovery of different genes involved in the disease propensity, very little is known about the involvement of genetics in the radiographic severity and/or radiographic progression of AS. Familial studies have demonstrated a high heritability, around 60% of radiographic severity of AS, suggesting a key role for genetics in this process (Brown *et al., supra,* Calin et al., 1989, Arthritis Rheum 1989; 32(1):77-81; Brophy et al., 2004, J Rheumatol; 31(9):1775-1778; Hamersma et al., 2001, Arthritis Rheum; 44(6):1396-1400). Revealing the genetic basis of the radiographic severity and/or radiographic progression of AS would be of great value to identify, at the moment of diagnosis, patients at high risk of developing severe radiographic damage or a fast radiographic progression of AS. This could allow clinicians to select and optimize the patients' preventive and therapeutic approach. Previous studies have shown that clinical factors may have prognostic significance in the radiographic status of the disease. Male sex, hip involvement and smoking status have been associated with worse radiographic condition (Averns et al., 1996, Scand J Rheumatol; 25(3): 138-142; Amor et al., 1994, J Rheumatol; 21(10):1883-1887; Lee et al., 2007; Ann Rheum Dis; 66(5):633-638; Pradeep et al., 2008; Rheumatology (Oxford); 47(7):942-945). However, few association studies have been performed to detect the specific genes associated with radiographic severity and/or radiographic progression of AS. In the case of single nucleotide polymorphisms (SNPs), the inventors are aware of only four studies, all of which have shown negative results. Specifically, SNPs at the *TNF-alpha, ERAP1, IL-23R* and *VEGF* genes showed no association with radiographic severity or radiographic progression of AS (Sousa et al., 2009, Ann. N Y Acad Sci; 1173:581-8; Pimentel-Santos et al., 2009, Clin Exp Rheumatol; 27:800-6; Seo et al., 2005, Rheumatology (Oxford); 44:1299-1302). In the case of the *VEGF* gene study, a single haplotype was found to be associated with radiographic severity of AS, but the association was lost after correction for disease duration (Seo *et al.,* 2005, *supra*). A recent study performed by Ward and collaborators has shown association of certain major histocompatibility complex (MHC) alleles only with the radiographic severity of spine in patients with AS (Ward et al., 2009, Arthritis Rheum; 61:859-66).

As a consequence, there is a need in the art for new markers for predicting the radiographic evolution of a subject suffering from AS which solve the problems of the prior art.

### SUMMARY OF THE INVENTION

The authors of the present invention have now found that some specific SNPs, either by themselves or in combinations among them, optionally in combination with one or more clinical variables, are highly and significantly associated with the radiographic evolution of AS in a subject suffering from AS and/or with the effective prediction of radiographic damage in AS patients.

Up to the inventors' knowledge, there is no a previous study which has demonstrated the association of SNPs with radiographic severity and/or radiographic progression of AS. The present invention demonstrates that one or more of the identified SNPs, optionally in combination with a clinical variable, allow for an effective prediction of radiographic damage in AS patients at early stages of the disease. Based on the results provided by the present invention, the clinician can optimize the patient's preventive and therapeutic care by choosing the most suitable therapeutic strategy depending on the predicted prognosis for each patient.

This information may allow physicians to follow patients more closely, as the present invention provides a method for predicting the radiographic clinical course of patients diagnosed of AS at the early stages of the disease. Some AS patients remain without significant radiographic changes for many years, while others become impaired within few years since disease onset. The methods and means of the present invention can help clinicians to distinguish between those AS patients who will progress very quickly towards a stage of severe radiographic damage from those patients who will remain without radiographic changes for years. Based on the results of the present invention, the clinician can optimize the patient's preventive and therapeutic care by choosing the most suitable therapeutic strategy from early stages of the disease and making informed decisions over treatment.

Thus, in an aspect, the invention relates to a method for predicting the risk of an AS patient to develop radiographic severity of AS, radiographic progression of AS, a condition that is associated with the radiographic severity of AS, and/or a condition that is associated with the radiographic progression of AS, which comprises detecting, in a sample from said patient, at least one SNP selected from the group consisting of the SNPs listed in Table 3, or a polymorphism in linkage diseq uilibrium (LD) with said at least one SNP, and predicting the risk for said AS patient of developing radiographic severity of AS, radiographic progression of AS, a condition that is associated with the radiographic severity of AS, and/or a condition that is associated with the radiographic progression of AS, based on the genotyping result previously obtained.

In another aspect, the invention relates to a method of classifying an AS patient as predisposed to develop radiographic severity of AS, radiographic progression of AS, a condition that is associated with the radiographic severity of AS, and/or a condition that is associated with the radiographic progression of AS, which comprises the steps of detecting, in a nucleic acid sample from said patient, at least one SNP selected from the group consisting of the SNPs listed in Table 3, or a polymorphism in LD with said at least one SNP, and classifying said AS patient as predisposed or not predisposed to develop radiographic severity of AS, radiographic progression of AS, a condition that is associated with the radiographic severity of AS, and/or a condition that is associated with the radiographic progression of AS, based on the genotyping result previously obtained.

In another aspect, the invention relates to a probe set, comprising a plurality of oligonucleotide probes that interrogate SNPs selected from those listed in Table 3, or polymorphisms in LD with said SNPs, wherein said oligonucleotide probes make up at least 50% of the oligonucleotide probes in the probe set.

### BRIEF DESCRIPTION OF THE DRAWINGS

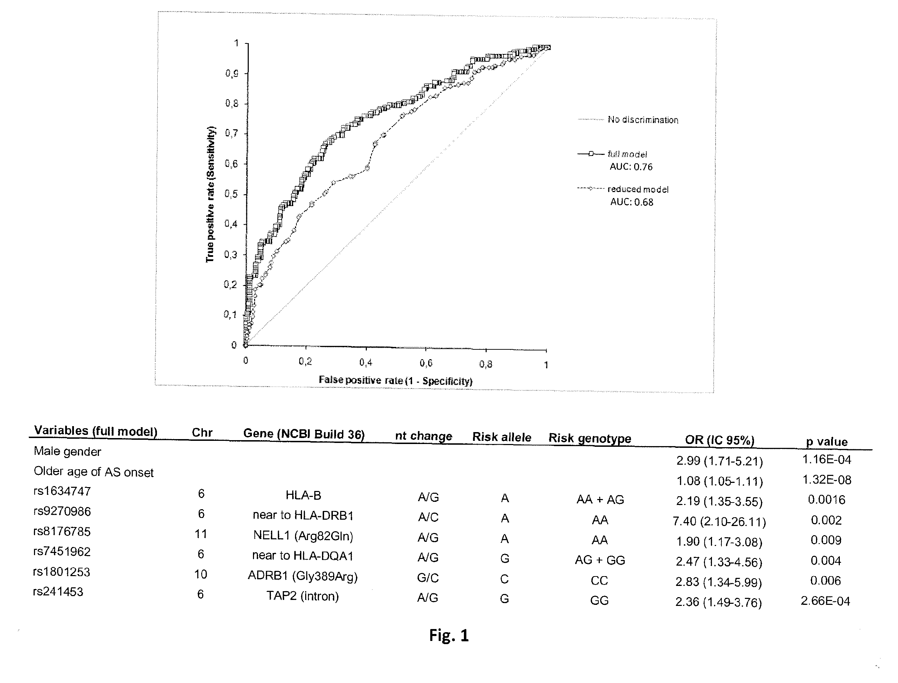

**Figure 1****. Predictive full model 1.** Multivariate logistic regression analysis to predict BASRI-t/duration (p60). The ROC curves of the full (SNPs plus clinical variables) and reduced model (clinical variables), the variables included in the full model (NCBI Build 36) and their OR (95% IC) and p-value are shown.

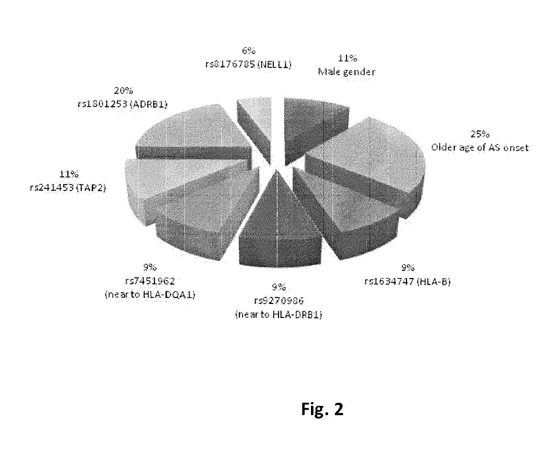

**Figure 2****.** Relative contribution of each variable to the predictive power of full model for BASRI-t/disease duration (p60).

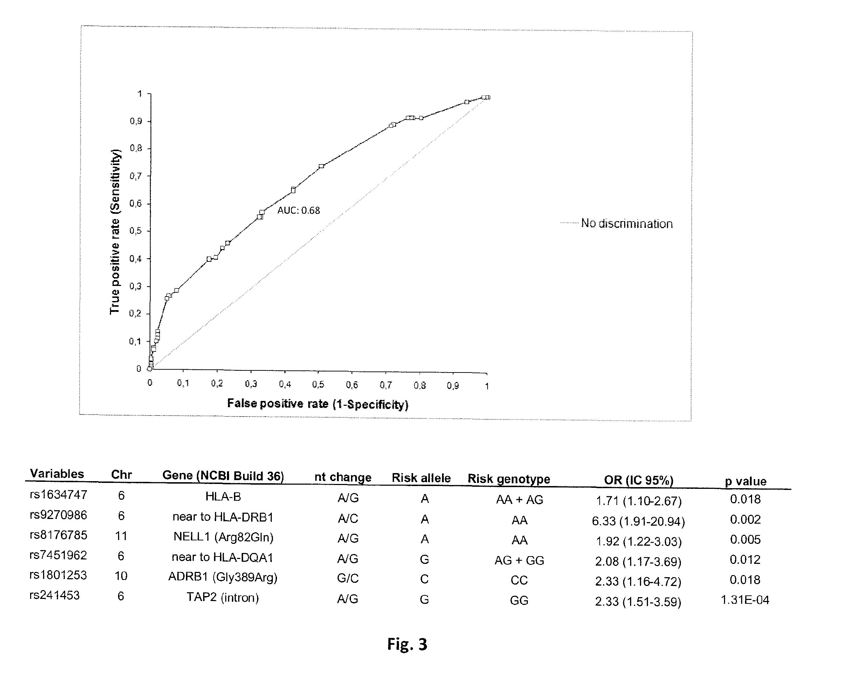

**Figure 3****. Predictive model 2.** Multivariate logistic regression analysis to predict BASRI-t/duration (p60). The ROC curve of a model formed by the combination of the 6 SNPs included in the full model of the example1, rs8176785, rs1634747, rs9270986, rs7451962, rs1801253 and rs241453 is shown.

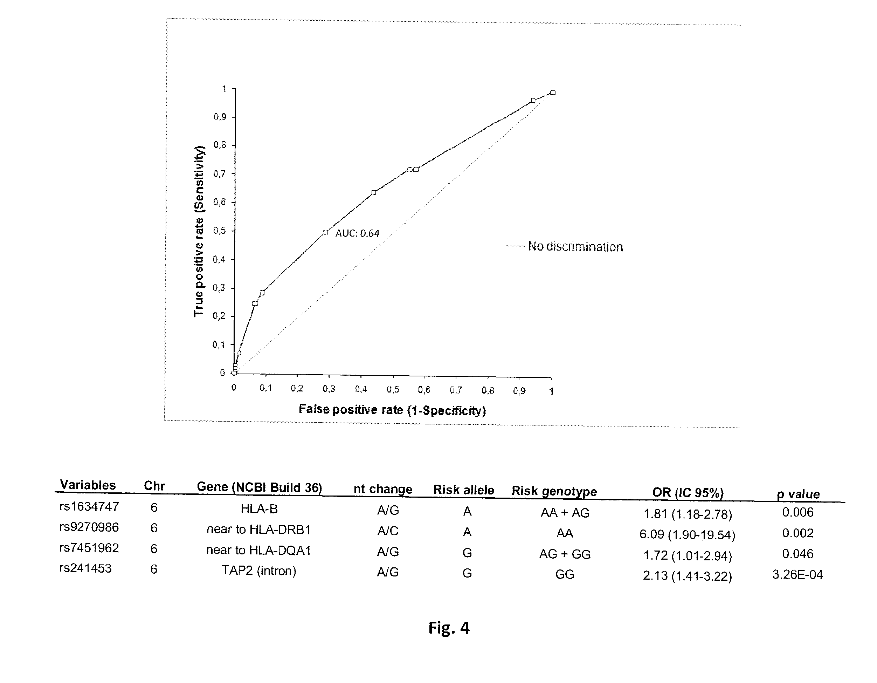

**Figure 4****. Predictive model 3.** Multivariate logistic regression analysis to predict BASRI-t/duration (p60). The ROC curve of a model formed by the combination of 4 of the SNPs included in the full model of example 1, which are all located within the MHC region: rs1634747, rs9270986, rs7451962 and rs241453, is shown.

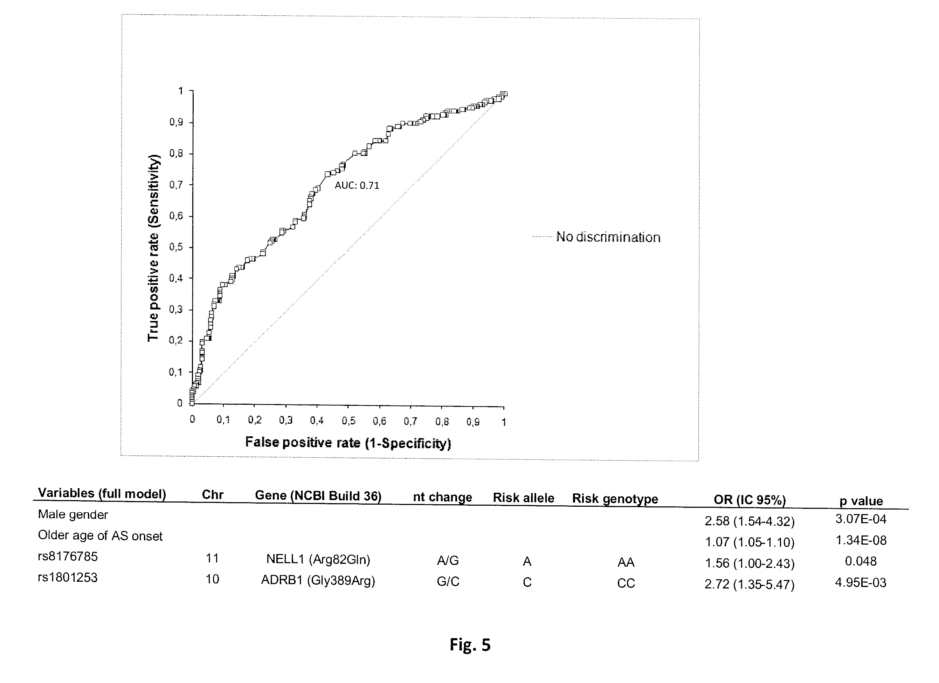

**Figure 5****. Predictive model 4.** Multivariate logistic regression analysis to predict BASRI-t/duration (p60). The ROC curve of a model including the 2 clinical variables and 2 of the SNPs of the full model of the example 1, rs1801253 and rs8176785, is shown.

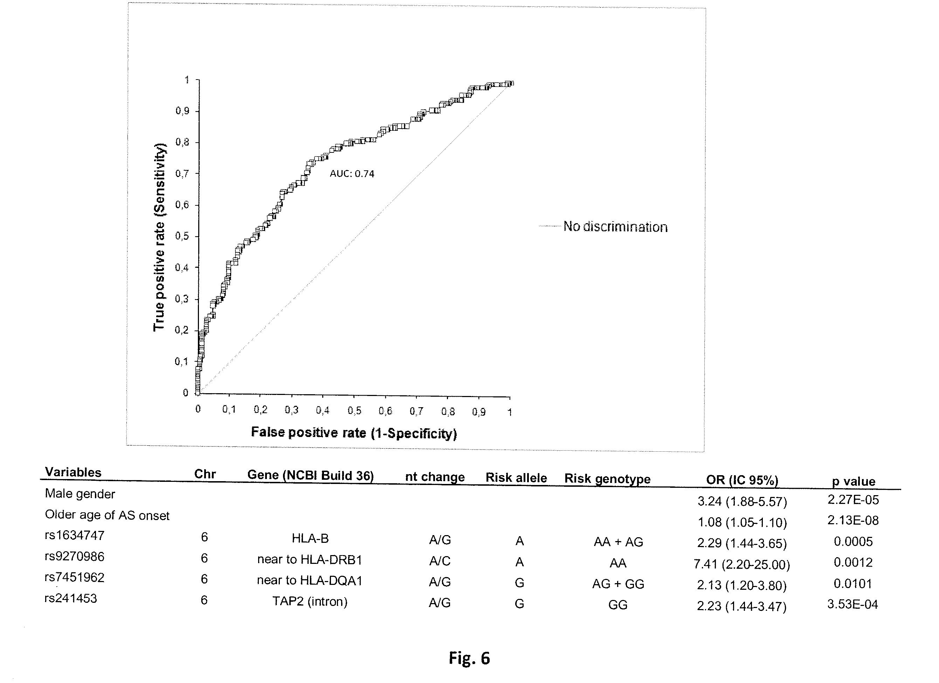

**Figure 6****. Predictive model 5.** Multivariate logistic regression analysis to predict BASRI-t/duration (p60). The ROC curve of a model including the 2 clinical variables and 4 of the SNPs of the full model of the example 1, which are all located inside the MHC region, rs1634747, rs9270986, rs7451962 and rs241453, is shown.

### DETAILED DESCRIPTION OF THE INVENTION

The authors of the present invention have found that the presence of specific polymorphisms either alone, or in combinations among them, and/or in combinations with clinical variables, can predict radiographic damage in AS patients.

The inventors performed a cross-sectional study where 473 AS patients were screened for 384 SNPs in 190 genes, and studied the association of the genotypes with radiographic severity and radiographic progression of AS. They found that the SNPs listed in Table 3, especially, SNPs rs1801253, rs8176785, rs1634747, rs9270986, rs7451962 and rs241453 were statistically significant. This finding opens the door to new genetic predictors of disease progression in the treatment of AS, helping the physician in the design of individualized therapy of AS patients. Based on these findings, the inventors have developed the methods of the present invention which will be described now in detail.

For the avoidance of doubt, the methods of the invention do not involve diagnosis practised on the human or animal body. The methods of the invention are preferably conducted on a sample that has previously been removed from the subject. The kits of the invention, described hereunder, may include means for extracting the sample from the subject.

### Definitions

The following definitions are set forth to illustrate and define the meaning and scope of various terms and expressions used to describe the invention therein.

The term "allele", as used herein, relates to one of two or more forms of a gene, locus or genetic polymorphism. Sometimes, different alleles can result in different traits; however, other times, different alleles will have the same result in the expression of a gene. Most multicellular organisms have two sets of chromosomes, that is, they are diploid. These chromosomes are referred to as homologous chromosomes. Diploid organisms have one copy of each gene (and one allele) on each chromosome. If both alleles are the same, they are homozygotes. If the alleles are different, they are heterozygotes.

The term "ankylosing spondylitis" or "AS", as used herein, refers to a subtype of spondyloarthritis with strong genetic predisposition. AS is a rheumatologic, inflammatory (autoimmune), progressive and chronic disease, that is characterized by chronic inflammation of the spine, hips and the sacroiliac joints, which causes pain and stiffness in and around the spine. Over time, this chronic spinal inflammation (spondylitis) leads to a partial or total fusion of the vertebrae, a process called ankylosis with the subsequent partial or total loss of mobility of the spine. Approximately 90% of AS patients have the HLA-B27 (human leukocyte antigen B27) genotype, meaning that there is a strong genetic association. However, only 5% of subjects with the HLA-B27 genotype contract the disease. A number of other factors are involved in AS, including male gender, Caucasian race, ERAP1 (endoplasmic reticulum aminopeptidase 1) and IL-23R (interleukin 23 receptor) variants. There is no direct test to diagnose AS. A combination of certain clinical criteria, including, but not limited to, low back pain and stiffness, limitation of motion of the lumbar spine and limitation of chest expansion, with evaluation of radiographs of the spine, which show characteristic spinal changes and sacroiliitis, are commonly used in the diagnosis of AS.

The term "AS patient", as used herein, refers to a subject suffering from AS, although the subject may have not been diagnosed yet.

The term "biomarker" or "marker", as used herein, refers to a genetic marker indicative of radiographic severity of AS, radiographic progression of AS, a condition that is associated with radiographic severity of AS, and/or a condition that is associated with radiographic progression of AS, that is to say a nucleic acid sequence which is specifically and significantly involved in the radiographic evolution of AS in a subject suffering from AS. In the context of the invention, such a marker may also be called a "risk marker".

The expression "condition that is associated with radiographic severity of AS", as used herein, relates to a condition that is commonly associated with radiographic severity of AS and includes, but is not restricted to, dactylitis, enthesitis, cervicalgy, tarsitis, sacroiliac syndrome, coxitis, low back pain, hip structural damage, osteopenia or osteoporosis of the spine (causing eventual compression fractures and a back "hump"), syndesmophytes, abnormal bone outgrowths similar to osteophytes affecting the spine, upper limbs arthritis, lower limbs arthritis, paresthesia, Achilles tendinitis, ectasia of the sacral nerve root sheaths, cauda equina syndrome, inflammation of the eye (iridocyclitis and uveitis, causing redness, eye pain, vision loss, floaters and photophobia), generalized fatigue, nausea, aortitis, apical lung fibrosis, aortic regurgitation, AV node block and amyloidosis, and other complications in the heart, colon and kidneys.

The expression "condition that is associated with the radiographic progression of AS", as used herein, relates to a condition that is commonly associated with radiographic progression of AS and includes, but is not restricted to, dactylitis, enthesitis, cervicalgy, tarsitis, sacroiliac syndrome, coxitis, low back pain, hip structural damage, osteopenia or osteoporosis of the spine (causing eventual compression fractures and a back "hump"), syndesmophytes, abnormal bone outgrowths similar to osteophytes affecting the spine, upper limbs arthritis, lower limbs arthritis, paresthesia, Achilles tendinitis, ectasia of the sacral nerve root sheaths,cauda equina syndrome, inflammation of the eye (iridocyclitis and uveitis, causing redness, eye pain, vision loss, floaters and photophobia), generalized fatigue, nausea, aortitis, apical lung fibrosis, aortic regurgitation, AV node block and amyloidosis, and other complications in the heart, colon and kidneys.

The term "haplotype", as used herein, refers to any combination of genetic markers. A haplotype can comprise two or more alleles. The haplotypes (or "at-risk haplotypes") described herein are found more frequently and significantly in AS patients at risk of developing radiographic severity of AS, radiographic progression of AS, a condition that is associated with radiographic severity of AS, and/or a condition that is associated with the radiographic progression of AS than in AS patients with decreased risk of developing radiographic severity of AS, radiographic progression of AS, a condition that is associated with radiographic severity of AS, and/or a condition that is associated with the radiographic progression of AS. Therefore, these haplotypes have predictive value for predicting the risk of AS patients of developing radiographic severity of AS, radiographic progression of AS, a condition that is associated with radiographic severity of AS, and/or a condition that is associated with the radiographic progression of AS, or a susceptibility to said conditions. An "at-risk haplotype" is thus intended to embrace one or a combination of haplotypes described herein over the markers that show high and significant correlation to risk of developing radiographic severity of AS, radiographic progression of AS, a condition that is associated with radiographic severity of AS, and/or a condition that is associated with the radiographic progression of AS.

The expression "linkage disequilibrium" or "LD", as used herein, refers to the non-random association of alleles at two or more loci. In other words, when some combinations of alleles or genetic markers at two or more loci in a population occur more or less often than expected from a random formation of haplotypes from alleles, based on their frequencies, said loci are in LD. Two polymorphisms are considered to be in linkage disequilibrium when the correlation coefficient of their allele frequencies (R²) is equal to or higher than 0.8 (R²≥0.8). The risk allele of a polymorphism in linkage disequilibrium with a SNP is the allele with a similar allelic frequency to the allelic frequency of the risk allele identified for the latter.

The term "polymorphism", as used herein, relates to a variation in the nucleotide sequence of a nucleic acid wherein each possible sequence is present in a proportion equal to or greater than a 1% of the population. In a specific case, when said variation in the nucleotide sequence occurs in a single nucleotide (A, C, T or G), it is referred to as "SNP" ("Single Nucleotide Polymorphism"). In the present invention, the terms polymorphism, polymorphic site and SNPs can be used indistinctly. The polymorphisms are typically named using the accession number in the SNP database (dbSNP) at National Center for Biotechnology Information (NCBI) accessible at http://www.ncbi.nlm.nih.gov/projects/SNP/. Alternatively, the polymorphism can be named as well by indicating the position at the gene or genomic contig wherein the variation occurs and the type of nucleotide change that occurs at said position or the type of amino acid change in the polypeptide encoding by said gene. When the nucleotide change corresponds with a change of amino acid in the protein, the polymorphism can be named as follows "aa1-nn-aa2", wherein "aa1" represents the original amino acid, "nn" corresponds to the position of the mutated amino acid, and "aa2" represents the amino acid resulting from the mutation. However, when the nucleotide change does not correspond with any change in the corresponding amino acid, then the polymorphism is named indicating the position of the nucleotide which contains the mutation and the nucleotide change is indicated as follows "mm nt1>nt2" wherein "mm" indicates the number of the nucleotide in which the change has occurred, "nt1" represents the original nucleotide and "nt2" the changed nucleotide. In general, SNPs represent one of the most common forms of genetic variations. These polymorphisms appear when a single nucleotide in the genome is altered (such as via substitution, addition or deletion). Each version of the sequence with respect to the polymorphic site is referred to as an allele of the polymorphic site. SNPs tend to be evolutionary stable from generation to generation and, as such, can be used to study specific genetic abnormalities throughout a population. If SNPs occur in the protein coding region, it can lead to the expression of a variant, sometimes defective, form of the protein that may lead to development of a genetic disease. Some SNPs may occur in non-coding regions, but nevertheless, may result in differential or defective splicing, or altered protein expression levels. SNPs can therefore serve as effective indicators of a genetic disease. SNPs can also be used as diagnostic and/or prognostic tools for identifying individuals with a predisposition for a disease or for a fast evolution of the disease, genotyping the individual suffering from the disease, and facilitating drug development based on the insight revealed regarding the role of target proteins in the pathogenesis process. In some embodiments of the present invention, the genetic markers used in the invention are particular alleles at "polymorphic sites" associated with radiographic severity of AS, radiographic progression of AS, a condition that is associated with radiographic severity of AS, and/or a condition that is associated with radiographic progression of AS. A nucleotide position in the genome at which more than one sequence is possible in a population is referred to as a "polymorphic site". Where a polymorphic site is a single nucleotide in length, the site is commonly called a SNP, as mentioned above; for example, if at a particular chromosomal location, one member of a population has an adenine (A) and another member of the population has a thymine (T) at the same position, then this position is a polymorphic site and, more specifically, the polymorphic site is an SNP. Polymorphic sites may be several nucleotides in length due to, e.g., insertions, deletions, conversions, substitutions, duplications, or translocations. Each version of the sequence with respect to the polymorphic site is referred to as an allele of the polymorphic site. Thus, in the previous example, the SNP allows for both an A allele and a T allele. These alleles are "variant" alleles. Nucleotide sequence variants, either in coding or in non-coding regions, can result in changes in the sequence of the encoded polypeptide, thus affecting the properties thereof (altered activity, altered distribution, altered stability, etc.). Alternatively, nucleotide sequence variants, either in coding or in non-coding regions, can result in changes affecting transcription of a gene or translation of its mRNA. In all cases, the alterations may be qualitative or quantitative or both.

The expression "predicting the risk" or "prediction of the risk", or similar, as used herein, is synonymous of the expression "assessing the risk" or "assessment of the risk", means that the present invention makes it possible to predict, estimate or evaluate the risk of an AS patient to develop radiographic severity of AS, radiographic progression of AS, a condition that is associated with radiographic severity of AS, and/or a condition that is associated with radiographic progression of AS (in this sense, this expression is also similar to "predisposition or susceptibility assessment"). In this respect, an AS patient "at risk" of developing radiographic severity of AS, radiographic progression of AS, a condition that is associated with radiographic severity of AS, and/or a condition that is associated with radiographic progression of AS is a patient who has at least one at-risk allele or haplotype with one or more SNPs associated to the development of radiographic severity of AS, radiographic progression of AS, a condition that is associated with radiographic severity of AS, and/or a condition that is associated with radiographic progression of AS. The prediction of risk generally implies that the risk is either increased or reduced. The risk of an AS patient who has at least one at-risk allele or haplotype with one or more SNPs associated to the development of radiographic severity of AS, radiographic progression of AS, a condition that is associated with radiographic severity of AS, and/or a condition that is associated with radiographic progression of AS is increased with respect to the risk of a patient who does not have said at-risk allele or haplotype with one or more SNPs associated to the development of radiographic severity of AS, radiographic progression of AS, a condition that is associated with radiographic severity of AS, and/or a condition that is associated with radiographic progression of AS.

In general, said expression ("predicting the risk", "prediction of the risk", or similar) relates to the likelihood that an AS patient will have a particular status of radiographic severity of AS, radiographic progression of AS, a condition that is associated with radiographic severity of AS, and/or a condition that is associated with radiographic progression of AS, either increased or decreased. As it will be understood by those skilled in the art, the prediction (or the risk), although preferred to be, need not be correct for 100% of the AS patients to be evaluated. The term, however, requires that a statistically significant portion of AS patients can be identified as having an increased probability of having a given status of radiographic severity of AS, radiographic progression of AS, a condition that is associated with radiographic severity of AS, and/or a condition that is associated with radiographic progression of AS. Whether a subject is statistically significant can be determined without further ado by the person skilled in the art by using various well known statistic evaluation tools, e.g., determination of confidence intervals, p-value determination, Student's t-test, Mann-Whitney test, etc. Details can be found in Dowdy and Wearden, Statistics for Research, John Wiley & Sons, New York 1983. Preferred confidence intervals are at least 50%, at least 60%, at least 70%, at least 80%, at least 90% at least 95%. The p-values are, preferably 0.1, 0.05, 0.02, 0.01 or lower.

The term "radiographic progression of AS", as used herein, relates to the evolution of AS in a patient over time, for example, between two time points. Progression of AS is different in each patient and can range from mild to progressively debilitating and from medically controlled to refractive. The progression of AS is measured on different levels, including:
(i) functional evolution, using specific questionnaires such as BASFI (Bath Ankylosing Spondylitis Functional Index),
(ii) activity evolution, using specific questionnaires such as BASDAI (Bath Ankylosing Spondylitis Disease Activity Index), and
(iii) structural evolution or radiographic severity, using specific indexes such as BASRI (Bath Ankylosing Spondylitis Radiology Index) and SASSS (Stoke Ankylosing Spondylitis Spine Score).

The term "radiographic severity of AS", as used herein, refers to the radiographic status of a patient in a specific time-point. The severity of AS varies from mild to severe. The radiographic status includes, but is not limited to the radiologic damage affecting the lumbar spine, lateral cervical spine, sacroiliac joints and hips. The level of severity can be established by using some specific indexes, such as those mentioned above, e.g., BASRI or SASSS, by the opinion of clinicians, etc.

The term "radiologic damage", as used herein, refers to any of the radiologic damage affecting the spine and joints, including, but not limited to lumbar spine, lateral cervical spine, sacroiliac joints and hips.

The term "risk allele", as used herein, refers to the allele that is associated with a certain condition; in this particular case, the risk allele is that allele that is associated with an increased risk for an AS patient to develop radiographic severity of AS, radiographic progression of AS, a condition that is associated with radiographic severity of AS, and/or a condition that is associated with radiographic progression of AS.

The term "risk genotype", as used herein, refers to both alleles residing at corresponding loci on homologous chromosomes that are associated with a certain condition. In the present invention, the terms risk genotype and high-risk genotype are used indistinctively and refer to the alleles associated to radiographic severity of AS, radiographic progression of AS, a condition that is associated with radiographic severity of AS, and/or a condition that is associated with radiographic progression of AS.

The term "sample", as used herein, relates to any sample which can be obtained from a subject. The methods of the present invention can be applied to any kind of biological sample from a subject, provided that said sample comprises a nucleic acid, such as a biopsy sample, a biological fluid (e.g., blood, cerebral spinal fluid (CSF), milk, mucus, plasma, saliva, semen, serum, sputum, sweat, synovial fluid, tears, urine, etc.), a cell, a tissue, etc. The samples can be obtained by conventional methods, e.g., swab, biopsy, etc., by using methods well known to those of ordinary skill in the related medical arts. Methods for obtaining the sample from the biopsy include gross apportioning of a mass, or microdissection, Tru-Cut biopsy or other art-known cell-separation methods. The cell or tissue sample from which the nucleic acid is prepared is typically taken from the subject under study, namely, an AS patient previously diagnosed (or still not diagnosed) with AS, or from an AS patient undergoing treatment, etc. Methods of isolating cell and tissue samples are well known to those skilled in the art and include, but are not limited to, aspirations, tissue sections, needle biopsies, and the like. A "nucleic acid sample", as used herein, refers to a sample comprising a nucleic acid representative of DNA or RNA isolated from a natural source. A nucleic acid sample, if desired, can be present in a form suitable for hybridization (e.g., as a soluble aqueous solution) to another nucleic acid (e.g., immobilized probes). The nucleic acid sample can be isolated, cloned, or extracted from particular cells or tissues. Frequently the sample will be a "clinical sample" which is a sample derived from an AS patient, including sections of tissues such as frozen sections or paraffin sections taken for histological purposes. The sample can also be derived from supernatants (of cells) or the cells themselves taken from the AS patients or from cell cultures, cells from tissue culture and the like. In some cases, the nucleic acids can be amplified, either directly from a biological fluid or tissue or after isolation, using standard techniques such as polymerase chain reaction (PCR), prior to genotyping.

The term "SNP genotyping", as used herein, refers to the determination of SNPs. The genotype, the result of one SNP in one subject is determined from a sample of the nucleic acid from the subject. The detection of the polymorphism can be performed by means of multiple processes known by the person skilled in the art.

The term "subject" or "individual", as used herein, includes any animal classified as a mammal and includes, but is not restricted to, domestic and farm animals, primates and humans, e.g., human beings, non-human primates, cows, horses, pigs, sheep, goats, dogs, cats or rodents. Preferably, the subject is a human being, male or female, of any age or race.

### Method for predicting the risk of an AS patient to develop radiographic severity of AS, radiographic progression of AS, a condition that is associated with radiographic severity of AS, and/or a condition that is associated with radiographic progression of AS

In an aspect, the invention relates to a method for predicting the risk of an ankylosing spondylitis (AS) patient to develop radiographic severity of AS, radiographic progression of AS, a condition that is associated with radiographic severity of AS, and/or a condition that is associated with radiographic progression of AS (hereinafter referred to as the "first method of the invention"), which comprises the steps of:
a) detecting, in a sample from said patient, at least one single nucleotide polymorphism (SNP) selected from the group consisting of the SNPs listed in Table 3, or a polymorphism in linkage disequilibrium (LD) with said at least one SNP, and
b) predicting the risk for said AS patient of developing radiographic severity of AS, radiographic progression of AS, a condition that is associated with radiographic severity of AS, and/or a condition that is associated with radiographic progression of AS, based on the genotyping result obtained in step a).

According to the first method of the invention, at least one SNP selected from the group consisting of the SNPs listed in Table 3, or a polymorphism in LD with said at least one SNP is detected in a sample from the AS patient. In a particular embodiment, the sample is a nucleic acid sample, i.e., a sample comprising a nucleic acid, e.g., DNA, genomic DNA (gDNA), complementary DNA (cDNA), RNA, heterogeneous nuclear RNA (hnRNA), mRNA, etc., from the patient under study. In an embodiment, the sample is isolated or extracted from a particular cell or tissue. The cells can be present in a biological fluid, e.g., blood, saliva, synovial fluid, etc., in a tissue, etc. In a particular embodiment, the sample is a biological fluid such as saliva, blood, synovial fluid or the like. Methods for isolating cell and tissue samples are well known to those skilled in the art and include, but are not limited to, aspirations, tissue sections, needle biopsies, and the like. In a particular embodiment, the sample is a clinical sample, as previously defined. In another embodiment, the sample derives from supernatants (of cells) or the cells themselves taken from the patients under study or from cell cultures, cells from tissue cultures and the like.

In some embodiments, for example, when the detection of the SNPs, or polymorphisms in LD with them, is carried out in a sample from whole blood, it may be used directly for the detection of said SNPs or polymorphisms in LD with them. In other embodiments, the nucleic acid is extracted from cells which are present in a biological fluid (e.g., whole blood, saliva, synovial fluid, etc.) as an initial step, and, in such cases, the total nucleic acid extracted from said samples would represent the working material suitable for subsequent amplification. Isolating the nucleic acid of the sample can be performed by methods known by the person skilled in the art. Said methods can be found, for example, in Sambrook et al., 2001. "Molecular cloning: a Laboratory Manual", 3rd ed., Cold Spring Harbor Laboratory Press, N.Y., Vol. 1-3. Further, as mentioned above, in some embodiments, generation of nucleic acids for analysis from samples requires nucleic acid amplification. Many amplification methods rely on an enzymatic chain reaction such as, for example, a polymerase chain reaction (PCR), a ligase chain reaction (LCR), or a self-sustained sequence replication, rolling-circle amplification assays, etc.; this list is merely illustrative and in no way limiting. Methods for amplifying nucleic acid are described in Sambrook *et al.,* 2001 (cited *at supra*).

After isolating, and amplifying (if necessary), the nucleic acid, the SNPs and/or polymorphisms in LD with them, are detected. Those skilled in the art will readily recognize that the analysis of the nucleotides present in one or several of the SNPs, or polymorphisms, disclosed herein in a patient's nucleic acid can be done by any method or technique capable of determining nucleotides present in a SNP or polymorphism. For instance, one may detect SNPs in the first method of the invention by performing sequencing, mini-sequencing, hybridization, restriction fragment analysis, oligonucleotide ligation assay, allele-specific PCR, or a combination thereof. As such, systems and methods for the detection of SNPs, polymorphic sites or polymorphisms, in general, include, but are not limited to, nucleic acid sequencing, hybridization methods and array technology (e.g. technology available from Aclara BioSciences, Affymetrix, Agilent Technologies, Illumina Inc., etc); also techniques based on mobility shift in amplified nucleic acid fragments, Single Stranded Conformational Polymorphism (SSCP), denaturing gradient gel electrophoresis (DGGE), Chemical Mismatch Cleavage (CMC), Restriction Fragment Polymorphisms (RFLPs), and WAVE analysis can be used (Methods Mol. Med. 2004; 108: 173-88), and the like. Of course, this list is merely illustrative and in no way limiting. Those skilled in the art may use any appropriate method to achieve such detection. As it is obvious in the art, the nucleotides present in said SNPs, polymorphic sites or polymorphisms, can be detected from either nucleic acid strand or from both strands.

Alternatively, the genotype of said AS patient could be detected by determining in a sample from said AS patient, the presence of a variant polypeptide encoded by a polynucleotide comprising a SNP listed in Table 3, or a polymorphism in LD with them. This is particularly suitable when the SNP involves a change in an amino acid, for example, in the SNP rs8176785 an Arg is replaced with a Gln in position 82 (Arg82Gln), in SNP rs1801253 a Gly is replaced with an Arg in position 389 (Gly389Arg), in SNP rs5789 a Leu is replaced with a Met in position 237 (Leu237Met), in SNP rs1800562 a Cys is replaced with a Tyr in position 282 (Cys282Tyr), in SNP rs11062385 a Met is replaced with a Thr in position 865 (Met865Thr), in SNP rs2294851 a Ser is replaced with an Asn in position 182 (Ser182Asn), in SNP rs3730089 a Met is replaced with an Ile in position 26 (Met26Ile), in SPN rs2071543 a Gln is replaced with a Lys in position 49 (Gln49Lys), in SNP rs26618 a Ile is replaced with a Met in position 276 (Ile276Met) and in SNP rs1805034 an Ala is replaced with a Val in position 192 (Ala192Val). The presence of said variant polypeptide can be determined by conventional methods, for example, by an immunoassay involving the formation of polypeptide-antibody complex by contacting the sample with an antibody that binds the variant polypeptide. Illustrative non-limiting examples of such immunoassays include, depending on the marker used, enzyme immunoassays, such as ELISA (enzyme-linked immunosorbent assay), fluoroimmunoassays, such as DELFIA (dissociation-enhanced lanthanide fluoroimmunoassay), luminescent immunoassays, radioimmunoassays such as RIA (heterogeneous competitive radioimmunoassay), IRMA (heterogeneous non-competitive radioimmunoassay), etc.

The SNPs to be detected according to the first method of the invention are selected from the group of SNPs listed in Table 3 below (see Example 1). In an embodiment, the first method of the invention comprises detecting in a nucleic acid sample from said patient at least one SNP selected from the 50 SNPs listed in Table 3. In a particular embodiment, the first method of the invention comprises detecting just one SNP selected from the SNPs listed in Table 3. In another particular embodiment, the first method of the invention comprises detecting two or more SNPs selected from the SNPs listed in Table 3, for example, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or, even, 50, SNPs listed in Table 3.

The SNPs listed in Table 3 are identified below:
- SNP rs1801253 is located in the *ADRB1* gene and corresponds to SEQ ID NO: 1;
- SNP rs8176785 is located in the *NELL1* gene and corresponds to SEQ ID NO: 2;
- SNP rs1634747 is located in the *HLA-B* gene (NCBI Build 36) and corresponds to SEQ ID NO: 3;
- SNP rs9270986 is located near to *HLA-DRB1* gene (NCBI Build 36) and corresponds to SEQ ID NO: 4;
- SNP rs7451962 is located near to *HLA-DQA1* gene (NCBI Build 36) and corresponds to SEQ ID NO: 5;
- SNP rs241453 is located in the *TAP2* gene and corresponds to SEQ ID NO: 6;
- SNP rs1573649 is located in the *HLA-DQB2* gene and corresponds to SEQ ID NO: 7;
- SNP rs11571300 is located near the *CTLA4* gene and corresponds to SEQ ID NO: 8;
- SNP rs4639334 is located in the *Major Histocompatibility Complex (MHC)* genes and corresponds to SEQ ID NO: 9;
- SNP rs5789 is located in the *PTGS1* gene and corresponds to SEQ ID NO: 10;
- SNP rs1801132 is located in the *ESR1* gene and corresponds to SEQ ID NO: 11;
- SNP rs10484554 is intergenic, between the *HLA-C* and the *HLA-B* genes, and corresponds to SEQ ID NO: 12;
- SNP rs2189480 is located in the *VDR* gene and corresponds to SEQ ID NO: 13;
- SNP rs6478108 is located in the *TNFSF15* gene and corresponds to SEQ ID NO: 14;
- SNP rs331377 is located in the *CENPP*/*ASPN* gene and corresponds to SEQ ID NO: 15;
- SNP rs1800562 is located in the *HFE* gene and corresponds to SEQ ID NO: 16;
- SNP rs11062385 is located in the *KDM5A*/*JARID1A* gene and corresponds to SEQ ID NO: 17;
- SNP rs615672 is located in the *Major Histocompatibility Complex (MHC)* genes and corresponds to SEQ ID NO: 18;
- SNP rs4246905 is located in the *TNFSF15* gene and corresponds to SEQ ID NO: 19;
- SNP rs7869487 is located in the *TNFSF15* gene and corresponds to SEQ ID NO: 20;
- SNP rs2301271 is located in the *Major Histocompatibility Complex (MHC)* genes and corresponds to SEQ ID NO: 21;
- SNP rs10865710 is located in the *PPARG* gene and corresponds to SEQ ID NO: 22;
- SNP rs2857210 is located in the *Major Histocompatibility Complex (MHC)* genes and corresponds to SEQ ID NO: 23;
- SNP rs2856997 is located in the *HLA-DOB* gene and corresponds to SEQ ID NO: 24;
- SNP rs2294851 is located in the *HHAT* gene and corresponds to SEQ ID NO: 25;
- SNP rs3811058 is located in the *IL1F10* gene and corresponds to SEQ ID NO: 26;
- SNP rs2235579 is located in the *CLCN7* gene and corresponds to SEQ ID NO: 27;
- SNP rs3730089 is located in the *PIK3R1* gene and corresponds to SEQ ID NO: 28;
- SNP rs6478109 is located in the *TNFSF15* gene and corresponds to SEQ ID NO: 29;
- SNP rs17034 is located in the *TAP2* gene and corresponds to SEQ ID NO: 30; - SNP rs8179673 is located in the *STAT4* gene and corresponds to SEQ ID NO: 31;
- SNP rs4263839 is located in the *TNFSF15* gene and corresponds to SEQ ID NO: 32;
- SNP rs10870077 is located in the *CARD9* gene and corresponds to SEQ ID NO: 33;
- SNP rs3783526 is located in the *IL1A* gene and corresponds to SEQ ID NO: 34;
- SNP rs3783543 is located in the *IL1A* gene and corresponds to SEQ ID NO: 35;
- SNP rs3783550 is located in the *IL1A* gene and corresponds to SEQ ID NO: 36;
- SNP rs3783546 is located in the *IL1A* gene and corresponds to SEQ ID NO: 37;
- SNP rs2542151 is located in the *PTPN2* gene and corresponds to SEQ ID NO: 38;
- SNP rs26618 is located in the *ERAP1* gene and corresponds to SEQ ID NO: 39;
- SNP rs7743761 is located in the *Major Histocompatibility Complex (MHC)* genes and corresponds to SEQ ID NO: 40;
- SNP rs2071543 is located in the *PSMB8* gene and corresponds to SEQ ID NO: 41;
- SNP rs724449 is located in the *PTH1R* gene and corresponds to SEQ ID NO: 42;
- SNP rs1049654 is located in the *CD36* gene and corresponds to SEQ ID NO: 43;
- SNP rs1533463 is located in the *IL1A* gene and corresponds to SEQ ID NO: 44;
- SNP rs1143627 is located in the *IL1B* gene and corresponds to SEQ ID NO: 45;
- SNP rs1217414 is located in the *PTPN22* gene and corresponds to SEQ ID NO: 46;
- SNP rs3025039 is located in the *VEGFA* gene and corresponds to SEQ ID NO: 47;
- SNP rs1805034 is located in the *TNFRSF11A* gene and corresponds to SEQ ID NO: 48;
- SNP rs2071482 is located in the *TAP1* gene and corresponds to SEQ ID NO: 49;
   and
- SNP rs4898 is located in the *TIMP1* gene and corresponds to SEQ ID NO: 50.

In a preferred embodiment, the SNP listed in Table 3 to be detected according to the first method of the invention is selected from the group of the SNPs rs1801253, rs8176785, rs1634747, rs9270986, rs7451962, rs241453 and any combination thereof. In a specific embodiment, the first method of the invention comprises detecting just one SNP selected from the SNPs rs1801253, rs8176785, rs1634747, rs9270986, rs7451962 and rs241453. In another specific embodiment, the first method of the invention comprises detecting two or more SNPs selected from the SNPs rs1801253, rs8176785, rs1634747, rs9270986, rs7451962 and rs241453, for example, 2, 3, 4, 5, or 6, of said SNPs. Said SNPs are of highly significant predictive value.

Alternatively, in a particular embodiment, the first method of the invention comprises detecting a polymorphic site associated with at least one SNP selected from the group of SNPs listed in Table 3. The term "associated with", as used herein, means that a particular polymorphic site is structurally and/or functionally associated with a particular SNP, i.e., said polymorphic site is either in the corresponding locus, or in close proximity thereto, and/or said polymorphic site interacts with or affects the corresponding gene or the expression product thereof. The polymorphic sites of the SNPs listed in Table 3 are identified below:
- the polymorphic site rs1801253 is located in the *ADRB1* gene and corresponds to position 27 in SEQ ID NO: 1;
- the polymorphic site rs8176785 is located in the *NELL1* gene and corresponds to position 27 in SEQ ID NO: 2;
- the polymorphic site rs1634747 is located in the *HLA-B* gene (NCBI Build 36) and the *HLA-B* genes and corresponds to position 27 in SEQ ID NO: 3;
- the polymorphic site rs9270986 is located near to *HLA-DRB1* gene (NCBI Build 36) and corresponds to position 27 in SEQ ID NO: 4;
- the polymorphic site rs7451962 is located near to *HLA-DQA1* gene (NCBI Build 36) and corresponds to position 27 in SEQ ID NO: 5;
- the polymorphic site rs241453 is located in the *TAP2* gene and corresponds to position 27 in SEQ ID NO: 6;
- the polymorphic site rs1573649 is located in the *HLA-DQB2* gene and corresponds to position 27 in SEQ ID NO: 7;
- the polymorphic site rs11571300 is located near the *CTLA4* gene and corresponds to position 27 in SEQ ID NO: 8;
- the polymorphic site rs4639334 is located in the *Major Histocompatibility Complex (MHC)* genes and corresponds to position 27 in SEQ ID NO: 9;
- the polymorphic site rs5789 is located in the *PTGS1* gene and corresponds to position 27 in SEQ ID NO: 10;
- the polymorphic site rs1801132 is located in the *ESR1* gene and corresponds to position 27 in SEQ ID NO: 11;
- the polymorphic site rs10484554 is intergenic, between the *HLA-C* and the *HLA-B* genes, and corresponds to position 27 in SEQ ID NO: 12;
- the polymorphic site rs2189480 is located in the *VDR* gene and corresponds to position 27 in SEQ ID NO: 13;
- the polymorphic site rs6478108 is located in the *TNFSF15* gene and corresponds to position 27 in SEQ ID NO: 14;
- the polymorphic site rs331377 is located in the *CENPP*/*ASPN* gene and corresponds to position 27 in SEQ ID NO: 15;
- the polymorphic site rs1800562 is located in the *HFE* gene and corresponds to position 27 in SEQ ID NO: 16;
- the polymorphic site rs11062385 is located in the *KDM5A*/*JARID1A* gene and corresponds to position 27 in SEQ ID NO: 17;
- the polymorphic site rs615672 is located in the *Major Histocompatibility Complex (MHC)* genes and corresponds to position 27 in SEQ ID NO: 18;
- the polymorphic site rs4246905 is located in the *TNFSF15* gene and corresponds to position 27 in SEQ ID NO: 19;
- the polymorphic site rs7869487 is located in the *TNFSF15* gene and corresponds to position 27 in SEQ ID NO: 20;
- the polymorphic site rs2301271 is located in the *Major Histocompatibility Complex (MHC)* genes and corresponds to position 27 in SEQ ID NO: 21;
- the polymorphic site rs10865710 is located in the *PPARG* gene and corresponds to position 27 in SEQ ID NO: 22;
- the polymorphic site rs2857210 is located in the *Major Histocompatibility Complex (MHC)* genes and corresponds to position 27 in SEQ ID NO: 23;
- the polymorphic site rs2856997 is located in the *HLA-DOB* gene and corresponds to position 27 in SEQ ID NO: 24;
- the polymorphic site rs2294851 is located in the *HHAT* gene and corresponds to position 27 in SEQ ID NO: 25;
- the polymorphic site rs3811058 is located in the *IL1F10* gene and corresponds to position 27 in SEQ ID NO: 26;
- the polymorphic site rs2235579 is located in the *CLCN7* gene and corresponds to position 27 in SEQ ID NO: 27;
- the polymorphic site rs3730089 is located in the *PIK3R1* gene and corresponds to position 27 in SEQ ID NO: 28;
- the polymorphic site rs6478109 is located in the *TNFSF15* gene and corresponds to position 27 in SEQ ID NO: 29;
- the polymorphic site rs17034 is located in the *TAP2* gene and corresponds to position 27 in SEQ ID NO: 30;
- the polymorphic site rs8179673 is located in the *STAT4* gene and corresponds to position 27 in SEQ ID NO: 31;
- the polymorphic site rs4263839 is located in the *TNFSF15* gene and corresponds to position 27 in SEQ ID NO: 32:
- the polymorphic site rs10870077 is located in the *CARD9* gene and corresponds to position 27 in SEQ ID NO: 33;
- the polymorphic site rs3783526 is located in the *IL1A* gene and corresponds to position 27 in SEQ ID NO: 34;
- the polymorphic site rs3783543 is located in the *ILIA1* gene and corresponds to position 27 in SEQ ID NO: 35;
- the polymorphic site rs3783550 is located in the *IL1A* gene and corresponds to position 27 in SEQ ID NO: 36;
- the polymorphic site rs3783546 is located in the *IL1A* gene and corresponds to position 27 in SEQ ID NO: 37;
- the polymorphic site rs2542151 is located in the *PTPN2* gene and corresponds to position 27 in SEQ ID NO: 38;
- the polymorphic site rs26618 is located in the *ERAP1* gene and corresponds to position 27 in SEQ ID NO: 39;
- the polymorphic site rs7743761 is located in the *Major Histocompatibility Complex (MHC)* genes and corresponds to position 27 in SEQ ID NO: 40;
- the polymorphic site rs2071543 is located in the *PSMB8* gene and corresponds to position 27 in SEQ ID NO: 41;
- the polymorphic site rs724449 is located in the *PTH1R* gene and corresponds to position 27 in SEQ ID NO: 42;
- the polymorphic site rs1049654 is located in the *CD36* gene and corresponds to position 27 in SEQ ID NO: 43;
- the polymorphic site rs1533463 is located in the *IL1A* gene and corresponds to position 27 in SEQ ID NO: 44;
- the polymorphic site rs1143627 is located in the *IL1B* gene and corresponds to position 27 in SEQ ID NO: 45;
- the polymorphic site rs1217414 is located in the *PTPN22* gene and corresponds to position 27 in SEQ ID NO: 46;
- the polymorphic site rs3025039 is located in the *VEGFA* gene and corresponds to SEQ ID NO: 47;
- the polymorphic site rs1805034 is located in the *TNFRSF11A* gene and corresponds to position 27 in SEQ ID NO: 48;
- the polymorphic site rs2071482 is located in the *TAP1* gene and corresponds to position 27 in SEQ ID NO: 49; and
- the polymorphic site rs4898 is located in the *TIMP1* gene and corresponds to position 27 in SEQ ID NO: 50.

In another embodiment, the first method of the invention comprises detecting in a nucleic acid sample from said patient at least one polymorphism in LD with any of said SNPs listed in Table 3. In a particular embodiment, the first method of the invention comprises detecting one or more polymorphisms in LD with one of the SNPs listed in Table 3. In another particular embodiment, the first method of the invention comprises detecting one or more polymorphisms in LD with two or more of the SNPs listed in Table 3. As mentioned above, linkage disequilibrium (LD) is the non-random association of alleles at two or more loci. In other words, when some combinations of alleles or genetic markers at two or more loci in a population occur more or less often than expected from a random formation of haplotypes from alleles, based on their frequencies, said loci are in LD. Two polymorphisms are considered to be in linkage disequilibrium when the correlation coefficient of their allele frequencies (R²) is equal to or higher than 0.8 (R²≥0.8). The risk alleles of the polymorphisms in LD with each one of the SNPs listed in Table 3 can be directly identified. The risk allele is the allele with a similar allelic frequency to the allelic frequency of the risk allele identified in this study. For example, the A allele is the risk allele of the SNP rs8176785 (Table 3), and, as it is described in the public database of the International HapMap Project (www.hapmap.org), the A allele shows an allelic frequency of 0.733 (f(A)=0.733) in Caucasian population. For example, the following polymorphisms are in LD with rs8176785 (R²≥0.8) (polymorphisms list not limited to): rs11825779, rs2280360, rs11025757, rs11821875, rs2280363, rs11025762, rs11823558, rs11025754, rs12365800, rs11025758, rs2280359, rs11025761 (polymorphisms set obtained from the public database of the International HapMap Project). The risk alleles of these polymorphisms in LD with rs8176785 are the following: the A allele in the SNP rs11825779 (f(A)=0.733), the C allele in the SNP rs2280360 (f(C)=0.724), the A allele in the SNP rs11025757 (f(A)=0.733), the G allele in the SNP rs11821875 (f(G)=0.733), the C allele in the SNP rs2280363 (f(C)=0.733), the C allele in the SNP rs11025762 (f(C)=0.692), the T allele in the SNP rs11823558 (f(T)=0.733), the C allele in the SNP rs11025754 (f(C)=0.767), the C allele in the SNP rs12365800 (f(C)=0.733), the C allele in the SNP rs11025758 (f(C)=0.733), the A allele in the SNP rs2280359 (f(A)=0.737), and the A allele in the SNP rs11025761 (f(A)=0.733).

Once at least one SNP selected from the group of SNPs listed in Table 3, or a polymorphism in LD with it, is detected, the first method of the invention further comprises predicting the risk for the AS patient to develop radiographic severity of AS, radiographic progression of AS, a condition that is associated with radiographic severity of AS, and/or a condition that is associated with radiographic progression of AS [step b)], based on the results obtained in step a). In this connection, the invention provides not only some specific SNPs which, by themselves or in combinations among them, and, optionally, in combination with one or more clinical variables, are significantly associated with the radiographic evolution of AS in a subject suffering from AS and/or with the effective prediction of radiographic damage in AS patients but also the corresponding risk alleles of each SNP which are mentioned in Table 3. Thus, in an embodiment, the first method of the invention further comprises determining the presence or absence of the risk alleles in the SNPs, or polymorphisms in LD with them, previously detected.

The risk alleles shown in Table 3 correspond to the TOP strand of the DNA following Ilumina's nomenclature for DNA strand identification. As it is known, the simplest case of determining strand designations occurs when one of the possible variations of the SNP is an adenine (A), and the remaining variation is either a cytosine (C) or guanine (G). In this instance, the sequence for this SNP is designated TOP. Similar to the rules of reverse complementarity, when one of the possible variations of the SNP is a thymine (T), and the remaining variation is either a C or a G, the sequence for this SNP is designated BOT. If the SNP is an [A/T] or a [C/G], then the above rules do not apply. Illumina employs a 'sequence walking' technique to designate the Strand for [A/T] and [C/G] SNPs. For this sequence walking method, the actual SNP is considered to be position 'n'. The sequences immediately before and after the SNP are 'n-1' and 'n+1', respectively. Similarly, two base pairs before the SNP is 'n-2' and two base pairs after the SNP 'n+2', etc. Using this method, sequence walking continues until an unambiguous pairing (A/G, A/C, T/C, or T/G.) is present. To designate strand, when the A or T in the first unambiguous pair is on the 5' side of the SNP, then the sequence is designated TOP. When the A or T in the first unambiguous pair is on the 3' side of the SNP, then the sequence is designated BOT.

According to the invention, once the genotype of at least one SNP selected from the group of SNPs listed in Table 3, or a polymorphism in LD with it, is determined, it is possible to establish if said at least one SNP contains the genotype corresponding to a risk allele (or not) for said SNP. Then, the prediction (or assessment) of the risk of radiographic severity of AS, radiographic AS, a condition that is associated with progression of radiographic severity of AS, and/or a condition that is associated with radiographic progression of AS, in the AS patient under study may be carried out based on the presence or absence of the corresponding risk allele for said at least one SNP, or polymorphism in LD with it, wherein:
- the presence of the risk allele in at least one allele of at least one of the SNPs listed in Table 3, is indicative of said AS patient having an increased risk of developing radiographic severity of AS, radiographic progression of AS, a condition that is associated with radiographic severity of AS and/or a condition that is associated with radiographic progression of AS; and/or
- the presence of the risk allele in at least one of said polymorphisms in LD with at least one of the SNPs listed in Table 3, is indicative of said patient having an increased risk of developing radiographic severity of AS, radiographic progression of AS, a condition that is associated with radiographic severity of AS and/or a condition that is associated with radiographic progression of AS.

The result of the prediction will be an increased or decreased risk of the subject under study to develop radiographic severity of AS, radiographic AS, a condition that is associated with progression of radiographic severity of AS, and/or a condition that is associated with radiographic progression of AS.

In a preferred embodiment, the SNP listed in Table 3 to be detected according to the first method of the invention is selected from the group of the SNPs rs1801253, rs8176785, rs1634747, rs9270986, rs7451962, rs241453 and any combination thereof. The risk alleles for said SNPs (which correspond to the TOP strand of Illumina's TOP-BOT nomenclature) are shown in Table 1.

**Table 1**

| **Risk alleles for SNPs rs1801253, rs8176785, rs1634747, rs9270986, rs7451962 and rs241453** | |
|---|---|
| **SNP** | **Risk allele** |
| rs1801253 | C |
| rs8176785 | A |
| rs1634747 | A |
| rs9270986 | A |
| rs7451962 | G |
| rs241453 | G |

Therefore, according to said embodiment, the presence of a risk allele in at least one allele of at least one of said SNPs rs1801253, rs8176785, rs1634747, rs9270986, rs7451962 and/or rs241453, such as those identified in Table 1is indicative of said AS patient having an increased risk of developing radiographic severity of AS, radiographic progression of AS, a condition that is associated with radiographic severity of AS and/or a condition that is associated with radiographic progression of AS.

Further, the inventors have also observed that when the AS patient shows in one or more of the different predictive SNPs according to the present invention the nucleotide indicative of increased risk (risk allele) in both alleles (i.e., the patient is homozygous for the SNP indicative of an increased risk (risk allele) of developing radiographic severity of AS, radiographic progression of AS, a condition that is associated with radiographic severity of AS and/or a condition that is associated with radiographic progression of AS), the risk of said patient of developing radiographic severity of AS, radiographic progression of AS, a condition that is associated with radiographic severity of AS and/or a condition that is associated with radiographic progression of AS is increased. Thus, in another preferred embodiment, the first method of the invention comprises detecting at least one SNP selected from the group of the SNPs rs1801253, rs8176785, rs1634747, rs9270986, rs7451962, rs241453 and any combination thereof, and determining the presence or absence of the risk allele in both alleles corresponding to said at least one SNP, wherein the presence of the risk allele in both alleles of said at least one SNP is indicative of an increased risk of developing radiographic severity of AS, radiographic progression of AS, a condition that is associated with radiographic severity of AS and/or a condition that is associated with radiographic progression of AS. Therefore, in a specific embodiment, the presence of :
- the risk allele in both alleles of the SNP rs1801253;
- the risk allele in both alleles of the rs8176785;
- the risk allele in both alleles of the SNP rs1634747;
- the risk allele in both alleles of the SNP rs9270986;
- the risk allele in both alleles of the SNP rs7451962; and/or
- the risk allele in both alleles of the SNP rs241453,
wherein the risk alleles for said SNPs are shown in Table 1 is indicative of said patient having an increased risk of developing radiographic severity of AS, radiographic progression of AS, a condition that is associated with radiographic severity of AS and/or a condition that is associated with radiographic progression of AS.

The above genotypes correspond to risk genotypes for developing radiographic severity of AS, radiographic progression of AS, a condition that is associated with radiographic severity of AS and/or a condition that is associated with radiographic progression of AS. Further risk genotypes identified by the inventors include:
- the presence of an A in one allele of the SNP rs1634747 and the presence of a G in the other allele of the rs1634747, and
- the presence of an A in one allele of the SNP rs7451962 and the presence of a G in the other allele of the rs7451962.

The predictive model based on one or more of the SNPs listed in Table 3, or polymorphisms in LD with them, has shown to have a value for predicting or assessing the risk of an AS patient to develop radiographic severity of AS, radiographic progression of AS, a condition that is associated with radiographic severity of AS and/or a condition that is associated with radiographic progression of AS. In a particular embodiment, the predictive model is based on the detection of a SNP listed in Table 3 selected from the group of the SNPs rs1801253, rs8176785, rs1634747, rs9270986, rs7451962, rs241453 and any combination thereof. In another particular embodiment, the predictive model is based on the detection of 4 of said 6 SNPs, namely SNPs rs1634747, rs9270986, rs7451962 and rs241453. In another particular embodiment, the predictive model is based on the detection of 2 of said 6 SNPs, namely SNPs rs8176785 and rs1801253. In another particular embodiment, the predictive model is based on the detection of the 6 SNPs rs1801253, rs8176785, rs1634747, rs9270986, rs7451962 and rs241453.

Further, the inventors have also observed that a model based on the SNPs identified in the present invention plus clinical variables can improve the predictive value for radiographic severity compared with the model based only on clinical variables, as shown in Example 1, wherein a significant 8% increase in the AUC was registered when comparing the full model [a combination of 6 SNPs (rs1801253, rs8176785, rs1634747, rs9270986, rs7451962 and rs241453) with 2 clinical variables (male gender and older age at disease onset)].

Therefore, in another preferred embodiment, the first method of the invention further comprises obtaining or determining one or more clinical variables associated with radiographic severity of AS, radiographic progression of AS, a condition that is associated with radiographic severity of AS and/or a condition that is associated with radiographic progression of AS. Illustrative, non-limitative, examples of said clinical variables include gender, age at disease onset, age at diagnosis, time of evolution of the disease, cervicalgy, enthesitis, dactylitis, tarsitis , sacroiliac syndrome, coxitis, low back pain, upper limbs arthritis, lower limbs arthritis, uveitis, number of clinical manifestations at disease onset, number of clinical manifestations at diagnosis, radiographic damage, presence of syndesmophytes, number of syndesmophytes, HLA-B27 positivity, smoking status, family history of spondyloarthropaties, and any combination thereof. In a particular embodiment, said clinical variable is male gender and/or older age at disease onset.

### Method for identifying a SNP haplotype associated with the risk of an AS patient to develop radiographic severity of AS, radiographic progression of AS, a condition that is associated with radiographic severity of AS, and/or a condition that is associated with radiographic progression of AS

In another aspect, the invention relates to a method for identifying a SNP haplotype associated with the risk of an AS patient to develop radiographic severity of AS, radiographic progression of AS, a condition that is associated with the radiographic severity of AS, and/or a condition that is associated with the radiographic progression of AS (hereinafter referred to as the "second method of the invention"), which comprises the steps of:
a) detecting, in a nucleic acid sample from said patient, at least one single nucleotide polymorphism (SNP) selected from the group consisting of the SNPs listed in Table 3, or a polymorphism in linkage disequilibrium (LD) with said at least one SNP, and
b) identifying said SNP haplotype in said AS patient, wherein said SNP haplotype comprises said at least one SNP, or a polymorphism in LD with said at least one SNP, detected in step a).

The particulars of the nucleic acid sample have been previously mentioned in connection with the first method of the invention as well as the particulars of the SNPs to be detected. In a particular embodiment, the SNP listed in Table 3 to be detected according to the second method of the invention is selected from the group of the SNPs rs1801253, rs8176785, rs1634747, rs9270986, rs7451962, rs241453 and any combination thereof.

Detecting haplotypes can be accomplished by methods well known in the art for detecting sequences at polymorphic sites, as those mentioned in connection with the first method of the invention.

### Method of classifying an AS patient as predisposed or not predisposed to radiographic severity of AS, radiographic progression of AS, a condition that is associated with radiographic severity of AS and/or a condition that is associated with radiographic progression of AS

In another aspect, the invention relates to a method of classifying an AS patient as predisposed to develop radiographic severity of AS, radiographic progression of AS, a condition that is associated with the radiographic severity of AS, and/or a condition that is associated with the radiographic progression of AS (hereinafter referred to as the "third method of the invention"), which comprises the steps of:
a) detecting, in a nucleic acid sample from said patient, at least one single nucleotide polymorphism (SNP) selected from the group consisting of the SNPs listed in Table 3, or a polymorphism in linkage disequilibrium (LD) with said at least one SNP, and
b) classifying said AS patient as predisposed or not predisposed to develop radiographic severity of AS, radiographic progression of AS, a condition that is associated with the radiographic severity of AS, and/or a condition that is associated with the radiographic progression of AS, based on the genotyping result obtained in step a).

The particulars of the nucleic acid sample have been previously mentioned in connection with the first method of the invention as well as the particulars of the SNPs to be detected. In a particular embodiment, the SNP listed in Table 3 to be detected according to the second method of the invention is selected from the group of the SNPs rs1801253, rs8176785, rs1634747, rs9270986, rs7451962, rs241453 and any combination thereof.

Detecting said SNPs or polymorphisms in LD with them can be accomplished by methods well known in the art for detecting sequences at polymorphic sites, as those mentioned in connection with the first method of the invention.

The classification of an AS patient as "predisposed" means that the patient is likely to be afflicted with or may be developing develop radiographic severity of AS, radiographic progression of AS, a condition that is associated with the radiographic severity of AS, and/or a condition that is associated with the radiographic progression of AS. The classification of a patient as "not predisposed" means that the patient is not likely to be afflicted with or may not be developing develop radiographic severity of AS, radiographic progression of AS, a condition that is associated with the radiographic severity of AS, and/or a condition that is associated with the radiographic progression of AS.

In an embodiment, the third method of the invention further comprises determining the presence or absence of the risk alleles in the SNPs, or polymorphisms in LD with them, previously detected. According to the invention, once the genotype of at least one SNP selected from the group of SNPs listed in Table 3 or a polymorphism in LD with it, is determined, it is possible to establish if said at least one SNP contains the genotype corresponding to a risk allele (or not) for said SNP and then classify the AS patient as a patient predisposed to develop radiographic severity of AS, radiographic progression of AS, a condition that is associated with radiographic severity of AS, and/or a condition that is associated with radiographic progression of AS.

According to the third method of the invention, an AS patient is classified as a patient predisposed to develop radiographic severity of AS, radiographic progression of AS, a condition that is associated with radiographic severity of AS, and/or a condition that is associated with radiographic progression of AS, based on the genotyping results for said SNPs in the patient under study, when it is detected:
- a risk allele in at least one allele of at least one of the SNPs listed in Table 3; and/or
- a risk allele in at least one of said polymorphisms in LD with at least one of the SNPs listed in Table 3.

In a preferred embodiment, the SNP listed in Table 3 to be detected according to the third method of the invention is selected from the group of the SNPs rs1801253, rs8176785, rs1634747, rs9270986, rs7451962, rs241453 and any combination thereof. In this case, an AS patient is classified as predisposed to develop radiographic severity of AS, radiographic progression of AS, a condition that is associated with radiographic severity of AS, and/or a condition that is associated with radiographic progression of AS, based on the genotyping results for said SNPs, when a risk allele in at least one allele of at least one of said SNPs rs1801253, rs8176785, rs1634747, rs9270986, rs7451962 and/or rs241453 is present, wherein the risk alleles for said SNPs are shown in Table 1.

In a further preferred embodiment, an AS patient is classified as predisposed to develop radiographic severity of AS, radiographic progression of AS, a condition that is associated with radiographic severity of AS, and/or a condition that is associated with radiographic progression of AS, based on the genotyping results for said SNPs, according to the third method of the invention, when:
- the risk allele is present in both alleles of the SNP rs1801253;
- the risk allele is present in both alleles of the rs8176785;
- the risk allele is present in both alleles of the SNP rs1634747;
- the risk allele is present in both alleles of the SNP rs9270986;
- the risk allele is present in both alleles of the SNP rs7451962; and/or
- the risk allele is present in both alleles of the SNP rs241453,
wherein the risk alleles for said SNPs are showin in Table 1.

Further risk genotypes identified by the inventors include:
- the presence of an A in one allele of the SNP rs1634747 and the presence of a G in the other allele of the rs1634747, and
- the presence of an A in one allele of the SNP rs7451962 and the presence of a G in the other allele of the rs7451962.

Depending on the predisposition of the AS patient to develop radiographic severity of AS, radiographic progression of AS, a condition that is associated with radiographic severity of AS and/or a condition that is associated with radiographic progression of AS, the physician could design an individualized therapy for the AS patient.

The term "therapy", as used herein, refers to a therapeutic treatment wherein the goal is to prevent or reduce an unwanted physiological change or disease, such as AS, especially, radiographic severity of AS, radiographic progression of AS, a condition that is associated with radiographic severity of AS and/or a condition that is associated with radiographic progression of AS. Beneficial or desired clinical results include, but not limiting, release of symptoms stabilized pathological state (specifically not deteriorated), retard in the disease's progression, improve of the pathological state and remission (both partial and total), both detectable and not detectable. The terms "treat" and "treatment" are synonyms of the term "therapy" and can be used without distinction along the present description. "Treatment" can mean also prolong quality of life compared to the one expected if the treatment is not applied.

No cure is known for AS, although treatments and medications are available to reduce symptoms and pain. There are four major types of treatments used to treat AS:
- Physiotherapy: effective in reducing the inflammation and pain, although the radiologic damage is not modified.
- Pain-relieving drugs come in two major classes: First, non-steroidal anti-inflammatory drugs, which reduce inflammation and pain. Second, opioid analgesics, which are very effective in alleviating the type of chronic pain commonly experienced by those suffering from AS.
- Disease-modifying antirheumatic drugs (DMARDs): used to reduce the immune system response through immunosuppression.
- TNFα blockers (antagonists): indicated for the treatment of and are effective immunosuppressants in AS.

The term "physiotherapy", as used herein, refers to identifying and maximizing quality of life and movement potential within the spheres of promotion, prevention, treatment and intervention, habilitation and rehabilitation. This encompasses physical, psychological, emotional, and social well being. Physiotherapeutic treatments include, but are not restricted to, joint and spine mobilization and/or manipulation, therapeutic exercise, neuromuscular reeducation, hot/cold packs, and electrical muscle stimulation (e.g., cryotherapy, iontophoresis, electrotherapy, etc.) are modalities often used. Preferred methods of physiotherapy include swimming and slow movement muscle extending exercises like stretching, yoga, climbing, taichi, pilates metod and the like.

The term "non-steroidal anti-inflammatory drugs (NSAIDs)", as used herein, refers to are drugs with analgesic and antipyretic (fever-reducing) effects and which have, in higher doses, anti-inflammatory effects. The term "non-steroidal" is used to distinguish these drugs from steroids, which, among a broad range of other effects, have a similar eicosanoid-depressing, anti-inflammatory action. As analgesics, NSAIDs are unusual in that they are non-narcotic. The most prominent members of this group of drugs are aspirin, ibuprofen, naproxen phenylbutazone, diclofenac, indomethacin, and COX-2 inhibitors.

The term "opioid analgesic", as used herein, refers to a chemical that works by binding to opioid receptors, which are found principally in the central and peripheral nervous system and the gastrointestinal tract. The analgesic effects of opioids are due to decreased perception of pain, decreased reaction to pain as well as increased pain tolerance. The side effects of opioids include sedation, respiratory depression, constipation and a strong sense of euphoria. Opioids can cause cough suppression, which can be both an indication for opioid administration and an unintended side effect. Opioid analgesics include, without limitation, morphine, codeine, thebaine, hydromorphone, hydrocodone, oxycodone, oxymorphone, desomorphine, nicomorphine, dipropanoylmorphine, benzylmorphine and ethylmorphine, buprenorphine, fentanyl, pethidine, methadone, tramadol, dextropropoxyphene.

The term "disease-modifying antirheumatic drugs (DMARDs)", as used herein, refers to a category of otherwise unrelated drugs defined by their use in rheumatoid arthritis to slow down disease progression. The term is often used in contrast to non-steroidal anti-inflammatory drug (which refers to agents that treat the inflammation but not the underlying cause) and steroids (which blunt the immune response but are insufficient to slow down the progression of the disease). DMARDs include, without limitation, cyclosporin, methotrexate, sulfasalazine, and corticosteroids.

The term "TNFα blockers", as used herein, refers to antagonists of TNFα that are used in the treatment of clinical problems associated with autoimmune disorders. In the treatment of AS, TNFα blockers improve the activity (BASDAI) and functionality (BASFI) and may be also effective in decreasing or slowing down AS radiographic damage in a long-term period (Schiotis et al., 2011, Ann. Rheum. Dis.; 70(Suppl3):339; Baraliakos et al., 2011, Ann. Rheum. Dis.; 70(Suppl3):344). TNFα blockers include, without limitation, etanercept, infliximab, certolizumab pegol, golimumab, adalimumab, pentoxifylline and bupropion.

### Kits of the invention

The present invention also contemplates the preparation of kits for use in accordance with the present invention. Suitable kits include various reagents for use in accordance with the present invention in suitable containers and packaging materials, including tubes, vials, and shrink-wrapped and blow-molded packages.

Materials suitable for inclusion in an exemplary kit in accordance with the present invention comprise one or more of the following: gene specific PCR primer pairs (oligonucleotides) that anneal to DNA or cDNA sequence domains that flank the genetic polymorphisms of interest (SNPs listed in Table 3 or polymorphisms in LD with them); reagents capable of amplifying a specific sequence domain in either genomic DNA or cDNA without the requirement of performing PCR; reagents required to discriminate between the various possible alleles in the sequence domains amplified by PCR or non-PCR amplification (e.g., restriction endonucleases, oligonucleotide that anneal preferentially to one allele of the polymorphism, including those modified to contain enzymes or fluorescent chemical groups that amplify the signal from the oligonucleotide and make discrimination of alleles more robust); reagents required to physically separate products derived from the various alleles (e.g. agarose or polyacrylamide and a buffer to be used in electrophoresis, HPLC columns, SSCP gels, formamide gels or a matrix support for MALDI-TOF); or even, reagents capable of identifying the polypeptides and variants thereof encoded by the corresponding polynucleotides comprising the SNPs listed in Table 3, or polymorphisms in LD with them, e.g, antibodies (including monoclonal, polyclonal or intact antibodies and fragments thereof as well as recombinant antibodies, etc.) capable of recognizing and binding to said polypeptides or variants thereof.

Specifically contemplated are kits comprising two or more polymorphism-specific or allele-specific oligonucleotides or oligonucleotide pairs, wherein each polymorphism-specific or allele-specific oligonucleotide or oligonucleotide pair is directed to one of the polymorphisms recited herein, namely the SNPs listed in Table 3 or polymorphisms in LD with them. It will be appreciated that in this context the term "directed to" means an oligonucleotide or oligonucleotide pair capable of identifying the allele present at the polymorphism.

By illustrative, the present invention contemplates a kit comprising a probe set, comprising a plurality of oligonucleotide probes that interrogate SNPs selected from those set forth in the first method of the invention (SNPs listed in Table 3 or polymorphisms in LD with them), wherein said oligonucleotide probes make up at least 50% of the oligonucleotide probes in the probe set. Thus, said probe set, comprising a plurality of oligonucleotide probes that interrogate SNPs selected from those listed in Table 3, or polymorphisms in LD with said SNPs, wherein said oligonucleotide probes make up at least 50% of the oligonucleotide probes in the probe set, constitutes an additional aspect of this invention.

In an embodiment, the kit comprises one or more polymorphism-specific or allele-specific oligonucleotides or oligonucleotide pairs directed to two or more of the above polymorphisms, while in another embodiment the kit comprises one or more polymorphism- specific or allele-specific oligonucleotides or oligonucleotide pairs directed to all of the polymorphisms listed in Table 3.

In another embodiment, the kit comprises one or more antibodies which recognize and bind to a polypeptide or a variant thereof encoded by a polynucleotide comprising a SNP listed in Table 3. This kit may be useful, for example, for determining the presence of a polypeptide variant encoded by a polynucleotide which comprises a SNP and involves a change in an amino acid.

### Uses

In another aspect, the invention relates to the use of at least one polymorphism, wherein said polymorphism is a SNP selected from the group of SNPs listed in Table 3, or a polymorphism in LD with said SNP, or a combination thereof, for:
- predicting the risk of an AS patient to develop radiographic severity of AS, radiographic progression of AS, a condition that is associated with radiographic severity of AS, and/or a condition that is associated with radiographic progression of AS; or for
- identifying a SNP haplotype associated with the risk of an AS patient to develop radiographic severity of AS, radiographic progression of AS, a condition that is associated with the radiographic severity of AS, and/or a condition that is associated with the radiographic progression of AS; or for
- classifying an AS patient as predisposed or not predisposed to develop radiographic severity of AS, radiographic progression of AS, a condition that is associated with the radiographic severity of AS, and/or a condition that is associated with the radiographic progression of AS.

In a particular embodiment, said SNP is selected from the group of SNPs consisting of SNPs rs1801253, rs8176785, rs1634747, rs9270986, rs7451962, rs241453 and any combination thereof.

### EXAMPLES

The invention is described by way of the following examples which are to be construed as merely illustrative and not limitative of the scope of the invention. The following methods were common to all the Examples.

### 1. METHODS

### 1.1 Study population

A cross-sectional association study was carried out with 473 AS patients. The patients, who fulfilled the Modified New York Criteria for AS diagnosis and had at least 10 years of follow-up from the first symptoms of the disease, were recruited from 25 hospitals.

To assess the disease structural damage of the patients, the total Bath AS Radiological Index (BASRI)-t score was used. The evaluation of the radiographs for assessment of the BASRI-t score was performed in each center by a previously trained expert rheumatologist. The baseline characteristics of the patients at the beginning of the disease were recovered. Specifically, clinical and demographic data, including gender, age at disease onset, family history of AS, initial symptoms of Spondyloarthropaties (SpA) (inflammatory low back pain, cervicalgy, enthesitis, dactylitis, tarsitis, sacroiliac syndrome, coxitis, lower limbs arthritis and upper limbs arthritis) and the number of initial symptoms of SpA were recorded, as possible prognostic indicators.

### HLA-B27 typing and SNP genotyping

Genomic DNA was isolated from saliva samples using the Oragene™ DNA Self-Collection kit (DNA Genotek Inc., Ottawa, Canada). All the samples were tested for the presence of the HLA-B27 allele by conventional PCR.

384 SNPs distributed in 190 genes were selected to be analyzed. SNP genotyping was performed using the Illumina GoldenGate genotyping platform (Illumina, Inc., San Diego, CA).

### Statistical Analysis

To standardize the measure of radiographic severity, the inventors used the BASRI-t index corrected for duration of AS considered from the onset of the first symptoms of the disease (BASRI-t/duration). Several association analyses with different criteria were performed in order to define the radiographic severity group. Specifically, the inventors, taking into consideration the opinion of clinicians, considered the patients in the top 75^{th} (p75), 70^{th} (p70), 65^{th} (p65) or 60^{th} (p60) percentile of BASRI-t/duration as having severe radiographic damage. The cut-off values for p60, p65, p70 and p75 of BASRI-t/duration were 0.362, 0.380, 0.410 and 0.447, respectively.

Pruning of the initial dataset with default parameters [exclusion of (i) SNPs with poor genotype cloud clustering, (ii) SNPs with call-rate <85%, and (iii) samples with call rate<85%] resulted in 456 samples and 344 SNPs.

Association test for allele and genotype frequencies with BASRI-t/duration was performed by the chi-square (χ²) Test. The magnitude of association was expressed as odds ratio (OR) with a 95% confidence interval (CI). Association test for clinical variables was performed by the χ² Test for categorical variables and by the unpaired T-test for continuous variables. Multivariate forward logistic regression models for predicting radiographic severity (75^{th}, 70^{th}, 65^{th} and 60^{th} percentile of BASRI-t /duration) were developed using as predictors the most significant SNPs and clinical variables, ranked by the associated p-value (p<0.1). When 2 SNPs were in linkage disequilibrium (R²>0.8), only the one with lowest p-value for allelic association was included in the multivariate analysis.

The inventors tested their models' discrimination via Hosmer-Lemeshow statistic and the receiver operating characteristic (ROC) curve with 95% confidence intervals (CI). The values of Sensitivity (Se), Specificity (Sp), Positive Predictive Value (PPV), and Negative Predictive Value (NPV) of the created models were calculated to measure the impact of the SNPs and clinical variables included in the models.

The model with the best fit was obtained for the BASRI-t/duration p60 and was internally validated by split-sample validation. The study data set was splitted (10 times) into one data set to train or built the model (training set: 75% of the population) and another data set to validate it (validation set: 25% of the population). The average of the 10 training and validation ROC AUC (area under curve) were compared by means of a Z test.

To analyze the predictive power added to the models by the SNPs, the inventors compared the AUC of the model based only on clinical variables with that of the model based on clinical variables and SNPs using the Analyse-it software v2.09 (Analyse-it Software Ltd., Leeds, UK).

To describe the relative contribution of the chi-square (χ²) value of the SNPs and the clinical variables to the radiographic severity models, the predictive model was calculated by eliminating one predictive factor (SNPs or clinical variables) at time. The reduced model χ² value was recorded. Finally, SNPs and clinical variables contribution was compared to the sum of all variables contribution to identify the relative contribution of each predictive factor (SNPs or clinical variables).

The statistical analysis was performed with the SPSS v15.0 (SPSS, Chicago, IL, USA) and the Helix TreeTM version 6.4.2 (Golden Helix Inc., Bozeman, Montana, USA) softwares.

### EXAMPLE 1

### Clinical and demographic variables associated to radiographic severity

The study cohort included 456 AS patients (348 males and 108 females) with a mean age at disease onset of 26.06 ± 9.1 (mean ± S.E.) years, and an average time of evolution, from disease onset, of 24.7 ± 10.1 years. The 84.9% of the patients were HLA-B27 positive and the 19.3% have family history of AS.

As a representative example of the association of the SNPs and demographic and clinical variables with BASRI-t severity, the data corresponding to BASRI-t/duration p60 are shown in Tables 1 and 2. The inventors observed that male gender, older age at disease onset, and cervicalgy at disease onset were strongly associated with radiographic severity when the patients were classified according to BASRI-t/duration p60 (Table 2) and to any of the rest of the percentiles, p65, p70 and p75. The number of clinical manifestations at disease onset was also found to confer risk for severity of BASRI-t/duration when we considered p60 (p=0.002), p65 (p=0.028) and p70 (p=0.029). A borderline association was found for inflammatory low back pain in the case of BASRI-t/duration p60 and p65 (p=0.081, and p=0.065, respectively), reaching this association the statistical significance for BASRI-t/duration p70 (p=0.005) and 75 (p=0.016).

**Table 2**

| **Clinical and demographic characteristics of the ankylosing spondylitis cohort (n=456)** | | | | |
|---|---|---|---|---|
| | | **AS patients classified by BASRI-t (p60)** | | |
| **Characteristics** | | **severe (n=188)** | **mild (n=268)** | **p-value** |
| Gender, males/females | | 160/28 | 188/80 | 0, 0002* |
| Age (years), mean ± SD | | 50.61 ± 10.2 | 51.01 ± 10.7 | 0.690 |
| Age at disease onset (years), mean ± SD | | 28.80 ± 9.85 | 24.09 ± 7.95 | 2.01x10⁻⁰⁸ * |
| Age at disease diagnosis (years), mean ± SD | | 35.35 ± 11.18 | 34.04 ± 11.45 | 0.233 |
| Time of evolution^{#} (years), mean ± SD | | 21.75 ± 8.25 | 26.92 ± 10.75 | 4.94x10⁻⁰⁸* |
| Disease duration^{†} (years), mean ± SD | | 15.38 ± 8.84 | 16.89 ± 10.75 | 0.120 |
| Family history of ankylosing spondylitis | | 38 (22.2%) | 50 (20.2%) | 0.679 |
| HLA-B27 positivity | | 152 (80.8%) | 234 (87.6%) | 0.047* |
| Clinical manifestations at disease onset: | | | | |
| | Cervicalgy | 44 (23.4%) | 19(7.1%) | 6.7x10⁻⁰⁷ * |
| | Enthesitis | 13 (6.9%) | 17 (6.3%) | 0.809 |
| | Dactylitis | 4 (2.1%) | 4 (1.5%) | 0.723 |
| | Tarsitis | 3 (1.7%) | 7(3.1%) | 0.525 |
| | Sacroiliac syndrome | 85 (45.2%) | 119 (44.4%) | 0.860 |
| | Coxitis | 6 (3.2%) | 4 (1.5%) | 0.330 |
| | Low back pain | 147 (78.2%) | 190 (70.9%) | 0.081 |
| | Upper limbs arthritis | 30 (16%) | 50 (18.7%) | 0.456 |
| | Lower limbs arthritis | 7 (3.7%) | 7 (2.6%) | 0.498 |
| | Uveitis | 44 (23.8%) | 68 (25.5%) | 0.683 |
| Number of clinical manifestations disease onset: | | | | 0.002* |
| 1 | | 89 (47.3%) | 154 (57.5%) | |
| 2 | | 61 (32.4%) | 86 (32.1%) | |
| 3 | | 28 (14.9%) | 22 (8.2%) | |
| 4 | | 7 (3.7%) | 5 (1.9%) | |
| 5 | | 2 (1.1%) | 1 (0.4%) | |
| 6 | | 1 (0.5%) | 0 (0%) | |
| BASRI-t, mean ± SD | | 10.84 ± 3.25 | 6.11 ± 3.64 | 2.13x10⁻³⁸ * |
| BASRI-t/duration of AS (years), mean ± SD | | 0.53 ± 0.16 | 0.22 ± 0.8 | 1.78x10⁻⁹⁷ * |

| | | | | |
|---|---|---|---|---|
| ^{#}Time of evolution refers to the period since the first symptoms of the disease. †Disease duration refers to the period since diagnosis. *p≤0.05. | | | | |

The results of association of the SNPs with AS radiographic severity are shown in Table 3. When the allele frequencies were compared, the inventors found 13 SNPs significantly (p≤0.05) associated with AS radiographic severity (BASRI-t p60) and other 20 SNPs with a significant association (p<0.1). When the comparison was made between genotypes, the inventors found 12 SNPs associated to radiographic severity of AS at p≤0.05 and other 21 SNPs with a p<0.1 association. In conclusion, considering the allelic and genotypic association tests, the inventors have found 50 SNPs with a significant association to AS total radiographic severity measured as BASRI-t/duration of AS (p60).

**Table 3**

| **SNPs with a significant (p≤0.05 or p<0.1) association to AS radiographic severity as indicated by the allelic and/or genotypic association tests.** Radiographic severity of AS was measured as BASRI-t/duration (p60). The p-values for the allelic and genotypic association tests and for the additive, dominant and recessive models are shown. Risk allele corresponds to the TOP strand of Illumina's TOP-BOT nomenclature. | | | | | | |
|---|---|---|---|---|---|---|
| | | **p-value** | | | | |
| **Marker** | **Risk allele** | **Alelle** | **Genoptype** | **Additive model** | **Dominant model** | **Recessive model** |
| rs1634747 | A | 1.0E-03 | 5.0E-03 | 1.0E-03 | 3.0E-03 | 0.03 |
| rs9270986 | A | 1.0E-03 | 5.0E-03 | 7.0E-03 | 0.05 | 1.0E-03 |
| rs1573649 | G | 4.0E-03 | 6.0E-03 | 4.0E-03 | 1.0E-03 | 0.22 |
| rs11571300 | A | 7.0E-03 | 0.03 | 0.01 | 0.02 | 0.04 |
| rs4639334 | G | 0.01 | 0.12 | 0.04 | 0.05 | 0.08 |
| rs5789 | C | 0.02 | 0.01 | 0.02 | 0.01 | 0.23 |
| rs1801132 | G | 0.03 | 0.05 | 0.04 | 0.16 | 0.01 |
| rs10484554 | A | 0.03 | 0.01 | 0.04 | 0.14 | 7.0E-03 |
| rs2189480 | C | 0.03 | 0.07 | 0.03 | 0.16 | 0.02 |
| rs8176785 | A | 0.03 | 0.11 | 0.03 | 0.04 | 0.24 |
| rs1801253 | C | 0.03 | 0.07 | 0.04 | 0.17 | 0.02 |
| rs6478108 | G | 0.04 | 0.12 | 0.04 | 0.09 | 0.11 |
| rs331377 | G | 0.04 | 0.06 | 0.04 | 0.02 | 0.41 |
| rs241453 | G | 0.05 | 4.0E-03 | 0.04 | 4.0E-03 | 0.54 |
| rs1800562 | G | 0.05 | 0.17 | 0.06 | 0.07 | 0.40 |
| rs11062385 | G | 0.05 | 0.09 | 0.05 | 0.11 | 0.07 |
| rs615672 | G | 0.06 | 0.11 | 0.05 | 0.04 | 0.38 |
| rs7451962 | G | 0.06 | 0.12 | 0.05 | 0.2 | 0.05 |
| rs4246905 | A | 0.06 | 0.15 | 0.05 | 0.11 | 0.12 |
| rs7869487 | G | 0.06 | 0.13 | 0.05 | 0.14 | 0.08 |
| rs2301271 | A | 0.06 | 0.18 | 0.07 | 0.07 | 0.31 |
| rs10865710 | C | 0.07 | 0.17 | 0.07 | 0.06 | 0.56 |
| rs2857210 | A | 0.07 | 0.20 | 0.07 | 0.10 | 0.24 |
| rs2856997 | C | 0.07 | 0.08 | 0.07 | 0.02 | 0.47 |
| rs2294851 | A | 0.07 | 0.21 | 0.07 | 0.08 | 0.39 |
| rs3811058 | G | 0.08 | 0.25 | 0.09 | 0.11 | 0.39 |
| rs2235579 | T | 0.08 | 0.17 | 0.09 | 0.06 | 0.40 |
| rs3730089 | G | 0.08 | 0.09 | 0.08 | 0.15 | 0.06 |
| rs6478109 | A | 0.08 | 0.22 | 0.08 | 0.10 | 0.29 |
| rs17034 | G | 0.09 | 0.01 | 0.08 | 6.0E-03 | 0.43 |
| rs8179673 | A | 0.09 | 0.21 | 0.08 | 0.08 | 0.50 |
| rs4263839 | A | 0.09 | 0.23 | 0.09 | 0.11 | 0.29 |
| rs10870077 | C | 0.09 | 0.06 | 0.09 | 0.02 | 0.86 |
| rs3783526 | G | 0.1 | 0.07 | 0.10 | 0.38 | 0.02 |
| rs3783543 | G | 0.1 | 0.05 | 0.10 | 0.43 | 0.01 |
| rs3783550 | A | 0.12 | 0.05 | 0.12 | 0.5 | 0.01 |
| rs3783546 | C | 0.13 | 0.05 | 0.12 | 0.52 | 0.01 |
| rs2542151 | C | 0.15 | 0.08 | 0.14 | 0.07 | 0.34 |
| rs26618 | G | 0.17 | 0.03 | 0.18 | 0.66 | 1.0E-3 |
| rs7743761 | A | 0.24 | 0.05 | 0.15 | 0.02 | 0.61 |
| rs2071543 | A | 0.24 | 0.05 | 0.24 | 0.11 | 0.15 |
| rs724449 | G | 0.24 | 0.09 | 0.24 | 0.05 | 0.76 |
| rs1049654 | C | 0.25 | 0.03 | 0.23 | 0.86 | 0.01 |
| rs1533463 | A | 0.3 | 0.08 | 0.29 | 0.88 | 0.02 |
| rs1143627 | G | 0.5 | 0.08 | 0.49 | 0.71 | 0.04 |
| rs1217414 | A | 0.51 | 0.01 | 0.5 | 0.58 | 6.0E-03 |
| rs3025039 | G | 0.64 | 0.03 | 0.65 | 0.23 | 0.06 |
| rs1805034 | A | 0.71 | 0.08 | 0.71 | 0.14 | 0.27 |
| rs2071482 | C | 0.75 | 0.08 | 0.75 | 0.77 | 0.04 |
| rs4898 | G | 0.85 | 0.08 | 0.88 | 0.55 | 0.37 |

### Model to predict radiographic severity

In order to search for a predictive model for total radiographic severity, the most associated SNPs (p<0.1 in the allele or genotype association tests) were entered together with the clinical and demographic variables associated to radiographic severity into multivariate forward logistic regression analysis. In all the cases, BASRI-t/duration p60, p65, p70 and p75, the inventors obtained a model with a ROC AUC above 0.75. The model with the best fit was obtained when the BASRI-t/duration p60 was used as the criteria to classify the patients in the severe or mild group. This model was validated by internal split-sample analysis. As a practical example, if a prediction in 10 years time considered, the inventors could predict at the initial stages of the disease which patients would have a future BASRI-t score higher than 4.47 in 10 years.

The validated model (p60) combined 6 SNPs with 2 clinical variables. Specifically, the SNP rs1801253 in the beta(1)-adrenergic receptor (*ADRB1*) gene, the SNP rs8176785 in the nel like 1 precursor (*NELL1*) gene, the SNP rs1634747 in the *HLA-B* gene, the SNP rs9270986 near *HLA-DRB1* gene, the rs7451962 near *HLA-DQA1* gene and the rs241453 in the *TAP2* gene and 2 clinical variables, male gender and older age at disease onset. The model had a predictive power, as indicated by the ROC AUC, of 0.76 (95% CI: 0.71-0.80) (Figure 1).

The model based on SNPs plus clinical variables (full model) significantly improved the predictive value for radiographic severity compared with the model based only on clinical variables (reduced model; p=0.0004). Specifically, a significant 8% increase in the AUC was registered when comparing the full model with the reduced model (Figure 1). The sensitivity, specificity, positive predictive value and negative predictive value of the full and reduced models at different points of the ROC curves confirm the significant improvement of the full model over the reduced models (Table 4). For example, the reduced model had a specificity of 54% for a sensitivity of 70%, while the specificity of the full model increased a 16% (specificity 70%) at the same value of sensitivity (70%) (Table 4).

**Table 4**

| **Predictive accuracy of the full model (clinical variables plus SNPs) and reduced model (clinical variables) for BASRI-t/disease duration (p60).** The area under the ROC curve (AUC), Sensitivity (Se), Specificity (Sp), Positive Predictive Value (PPV), and Negative Predictive Value (NPV) are shown. | | | | | |
|---|---|---|---|---|---|
| | | | | | **NPV** |
| **Model** | **AUC** | **Se (%)** | **Sp (%)** | **PPV (%)** | **(%)** |
| Full model | 0.76 | 90 | 31 | 46 | 83 |
| | | 80 | 53 | 53 | 81 |
| | | 70 | 70 | 60 | 78 |
| | | 40 | 90 | 72 | 40 |
| Reduced model | 0.68 | 90 | 25 | 46 | 77 |
| | | 80 | 44 | 50 | 75 |
| | | 70 | 54 | 52 | 72 |
| | | 40 | 82 | 63 | 66 |

This model (predictive model 1) predicted total radiographic damage in AS, measured as BASRI-t/duration, and includes 6 SNPs and 2 clinical variables (Figure 1). Namely, the six SNPs are: rs8176785 (R82Q), located in *NELL1* gene; rs1801253 (G389R), located in the *ADRB*1 gene; rs9270986, rs1634747, rs7451962 and rs241453 are located in the *MHC* genes. The two clinical variables are male sex and older age at disease onset.

Of the six SNPs, two are non-MHC SNPs. Namely, rs8176785 (R82Q) is located in *NELL1* gene, which encodes a newly identified potent osteoinductive protein (Zhang et al., 2010, J Dent Res; 89(9):865-878; Lu et al., 2007, Spine J; 7(1):50-60). The second SNP is rs1801253 (G389R), located in the *ADRB*1 gene. The *ADRB*1 gene is mostly known for its role in the regulation of cardiac output, peripheral resistance and obesity. However, in the last years, several studies have suggested a role for adrenergic signaling in the regulation of bone remodeling (Bonnet et al., 2008, J Musculoskelet Neuronal Interact; 8(2):94-104; Pierroz et al., 2006, Bone; 39(2):260-267). The relative contribution of each genetic and clinical variable to the predictive power of the full model is depicted in Figure 2. Older age at disease onset and the SNP rs1801253 of the *ADRB1* gene are the variables with a highest contribution to the predictive power of the model, 25% and 20% respectively. In general, the clinical variables contribute 36% to the explained variability of the full model, being the remaining 64% explained by the genetic variables.

Four of the six SNPs included in the predictive model for BASRI-t (rs1634747, rs9270986 and rs7451962 in the major histocompatibility complex, and rs241453 in the *TAP2* gene of the major histocompatibility complex) were found to be also significantly (p<0.1) associated to severe AS radiographic damage of the spine (BASRI-s) (Table 5).

Three of the six SNPs included in the predictive model for BASRI-t (rs1801253 in the ADRB1 gene, rs9270986 in the major histocompatibility complex, and rs241453 in the *TAP2* gene of the major histocompatibility complex) were found to be also significantly (p<0.1) associated to severe AS radiographic damage of the hips (BASRI-h) (Table 6).

**Table 5**

| **Association of the 6 SNPs of the predictive model for total radiographic severity (BASRI-t) with AS radiographic severity of the spine (BASRI-s).** | | | | | |
|---|---|---|---|---|---|
| | BASRI-s (p-value) | | | | |
| **Marker** | **Allele** | **Genotype** | **Additive model** | **Dominant model** | **Recessive model** |
| rs1634747 | 0.001 | 0.005 | 0.001 | 0.004 | 0.022 |
| rs9270986 | 0.067 | 0.142 | 0.117 | 0.298 | 0.048 |
| rs8176785 | 0.413 | 0.309 | 0.4 | 0.244 | 0.985 |
| rs7451962 | 0.059 | 0.143 | 0.053 | 0.16 | 0.079 |
| rs1801253 | 0.035 | 0.092 | 0.04 | 0.119 | 0.05 |
| rs 241453 | 0.364 | 0.167 | 0.359 | 0.121 | 0.574 |

**Table 6**

| **Association of the 6 SNPs of the predictive model for total radiographic severity (BASRI-t) with AS radiographic severity of the hips (BASRI-h).** | | | | | |
|---|---|---|---|---|---|
| | BASRI-h (p-value) | | | | |
| **Marker** | **Allele** | **Genotype** | **Additive model** | **Dominant model** | **Recessive model** |
| rs1634747 | 0.238 | 0.437 | 0.244 | 0.350 | 0.269 |
| rs9270986 | 0.006 | 0.011 | 0.029 | 0.124 | 0.003 |
| rs8176785 | 0.232 | 0.461 | 0.222 | 0.270 | 0.416 |
| rs7451962 | 0.42 | 0.259 | 0.407 | 0.938 | 0.124 |
| rs1801253 | 0.016 | 0.047 | 0.018 | 0.070 | 0.030 |
| rs241453 | 0.054 | 0.004 | 0.051 | 0.003 | 0.556 |

The next examples show the power of prediction of different predictive models for BASRI-t/disease duration (p60) formed by specific combinations of some of the SNPs or SNPs plus clinical variables included in the full model.

### EXAMPLE 2

### Predictive model 2

The predictive power of a model formed by the combination of the 6 SNPs included in the full model of the Example 1 [rs8176785 (R82Q); rs1801253 (G389R); rs9270986; rs1634747; rs7451962 and rs241453] is shown in Figure 3. The power of prediction is 0.68 as indicated by the area under the ROC curve.

### EXAMPLE 3

### Predictive model 3

The predictive power of a model formed by the combination of 4 of the SNPs included in the full model of the Example 1, namely rs9270986; rs1634747; rs7451962 and rs241453, is shown in Figure 4. The power of prediction is 0.64 as indicated by the area under the ROC curve.

### EXAMPLE 4

### Predictive model 4

The predictive power of a model formed by the combination of the 2 clinical variables [male gender and older age of AS onset] and 2 of the SNPs [rs8176785 (R82Q); and rs1801253 (G389R)] of the full model of the Example 1 is shown in Figure 5. The power of prediction is 0.71 as indicated by the area under the ROC curve.

### EXAMPLE 5

### Predictive model 5

The predictive power of a model formed by the combination of the 2 clinical variables [male gender and older age of AS onset] and 4 of the SNPs [rs9270986; rs1634747; rs7451962 and rs241453] of the full model of the Example 1, which are all located in the MHC region, is shown in Figure 6. The power of prediction is 0.74 as indicated by the area under the ROC curve.

## Claims

1. A method for predicting the risk of an ankylosing spondylitis (AS) patient to develop radiographic severity of AS, radiographic progression of AS, a condition that is associated with the radiographic severity of AS, and/or a condition that is associated with the radiographic progression of AS, which comprises the steps of:
a) detecting, in a sample from said patient, at least one single nucleotide polymorphism (SNP) selected from the group consisting of the SNPs listed in Table 3, or a polymorphism in linkage disequilibrium (LD) with said at least one SNP, and
b) predicting the risk for said AS patient of developing radiographic severity of AS, radiographic progression of AS, a condition that is associated with the radiographic severity of AS, and/or a condition that is associated with the radiographic progression of AS, based on the genotyping result obtained in step a).

2. A method of classifying an AS patient as predisposed to develop radiographic severity of AS, radiographic progression of AS, a condition that is associated with the radiographic severity of AS, and/or a condition that is associated with the radiographic progression of AS, which comprises the steps of:
a) detecting, in a nucleic acid sample from said patient, at least one single nucleotide polymorphism (SNP) selected from the group consisting of the SNPs listed in Table 3, or a polymorphism in linkage disequilibrium (LD) with said at least one SNP, and
b) classifying said AS patient as predisposed or not predisposed to develop radiographic severity of AS, radiographic progression of AS, a condition that is associated with the radiographic severity of AS, and/or a condition that is associated with the radiographic progression of AS, based on the genotyping result obtained in step a).

3. Method according to any of claims 1 or 2, wherein the sample is selected from a biological fluid, a cell or a tissue.

4. Method according to any of claims 1 to 3, wherein the SNP is detected by performing sequencing, mini-sequencing, hybridization, restriction fragment analysis, oligonucleotide ligation assay, allele-specific PCR, or a combination thereof.

5. Method according to any of claims 1 to 3, wherein the SNP is detected by determining in a sample from said AS patient, the presence of a variant polypeptide encoded by a polynucleotide comprising a SNP listed in Table 3, or a polymorphism in LD with them.

6. Method according to claim 5, wherein the variant polypeptide is determined by an immunoassay which comprises contacting the sample with an antibody that binds the variant polypeptide.

7. Method according to any of claims 1 to 3, wherein the SNP to be detected is selected from the group of the SNPs rs1801253, rs8176785, rs1634747, rs9270986, rs7451962, rs241453 and any combination thereof, or a polymorphism in LD with said SNPs.

8. Method according to claim 1, which further comprises determining the presence or absence of the risk allele in at least one allele of at least one of the SNPs listed in Table 3, or in a polymorphism in LD with it, wherein
- the presence of the risk allele in at least one allele of at least one of the SNPs listed in Table 3, is indicative of said AS patient having an increased risk of developing radiographic severity of AS, radiographic progression of AS, a condition that is associated with radiographic severity of AS and/or a condition that is associated with radiographic progression of AS; and/or
- the presence of the risk allele in at least one of said polymorphisms in LD with at least one of the SNPs listed in Table 3, is indicative of said patient having an increased risk of developing radiographic severity of AS, radiographic progression of AS, a condition that is associated with radiographic severity of AS and/or a condition that is associated with radiographic progression of AS.

9. Method according to claim 8, wherein the presence of a risk allele in at least one allele of at least one SNP selected from the group of SNPs consisting of rs1801253, rs8176785, rs1634747, rs9270986, rs7451962, rs241453 and any combination thereof, wherein the risk allele for said at least one SNP is shown in Table 1,
is indicative of said AS patient having an increased risk of developing radiographic severity of AS, radiographic progression of AS, a condition that is associated with radiographic severity of AS and/or a condition that is associated with radiographic progression of AS.

10. Method according to claim 1, wherein the presence of:
the risk allele in both alleles of the SNP rs1801253;
the risk allele in both alleles of the rs8176785;
the risk allele in both alleles of the SNP rs1634747;
the risk allele in both alleles of the SNP rs9270986;
the risk allele in both alleles of the SNP rs7451962; and/or
the risk allele in both alleles of the SNP rs241453,
wherein the risk alleles for said SNPs are shown in Table 1 is indicative of said patient having an increased risk of developing radiographic severity of AS, radiographic progression of AS, a condition that is associated with radiographic severity of AS and/or a condition that is associated with radiographic progression of AS.

11. Method according to claim 1, which further comprises obtaining or determining one or more clinical variables associated with radiographic severity of AS, radiographic progression of AS, a condition that is associated with radiographic severity of AS and/or a condition that is associated with radiographic progression of AS.

12. Method according to claim 11, wherein said clinical variable comprises gender, age at disease onset, age at diagnosis, time of evolution of the disease, cervicalgy, enthesitis, dactylitis, tarsitis , sacroiliac syndrome, coxitis, low back pain, upper limbs arthritis, lower limbs arthritis, uveitis, number of clinical manifestations at disease onset, number of clinical manifestations at diagnosis, radiographic damage, presence of syndesmophytes, number of syndesmophytes, HLA-B27 positivity, smoking status, family history of spondyloarthropaties, and any combination thereof.

13. The method according to any of claims 1 to 3, further comprising selecting a therapy for said AS patient.

14. A probe set, comprising a plurality of oligonucleotide probes that interrogate SNPs selected from those set forth in claim 1, wherein said oligonucleotide probes make up at least 50% of the oligonucleotide probes in the probe set.


SEQUENCE LISTING

<110>  PROGENIKA BIOPHARMA, S.A.

<120>  METHOD FOR PREDICTING RADIOGRAPHIC SEVERITY IN ANKYLOSING
       SPONDYLITIS

<130>  P7134EP00

<160>  50

<170>  PatentIn version 3.5

<210>  1
<211>  52
<212>  DNA
<213>  Homo sapiens

<400>  1
gccccgactt ccgcaaggcc ttccagsgac tgctctgctg cgcgcgcagg gc          52

<210>  2
<211>  52
<212>  DNA
<213>  Homo sapiens

<400>  2
gagtgagaaa ttaattcagc tgttccrgaa caagagtgaa ttcaccattt tg          52

<210>  3
<211>  52
<212>  DNA
<213>  Homo sapiens

<400>  3
aaaattacaa gcaatgctgt tacaagyctt ctagcactag tctgttggca ca          52

<210>  4
<211>  52
<212>  DNA
<213>  Homo sapiens

<400>  4
agtaagaaat tactgggcat aactagmagg tgccatggga tgtgcctgga aa          52

<210>  5
<211>  52
<212>  DNA
<213>  Homo sapiens

<400>  5
tttatttcta ggaagggcag gaattcrgaa tccctcatcc tgtccccagc ta          52

<210>  6
<211>  52

<212>  DNA
<213>  Homo sapiens

<400>  6
accatagttg ctattttgct gcctgtycca tcagtcgttt atctgttgtt tg          52


<210>  7
<211>  52
<212>  DNA
<213>  Homo sapiens

<400>  7
cactgctttt tccttggtct cacttaygtc ttggaagatg gctctgcaga tc          52


<210>  8
<211>  52
<212>  DNA
<213>  Homo sapiens

<400>  8
cctctttaaa ggtttgggta gacaaayatg gtaagcactc tctcattgcc ca          52


<210>  9

<400>  9
000

<210>  10
<211>  52
<212>  DNA
<213>  Homo sapiens

<400>  10
acctcggcca catttatgga gacaatmtgg agcgtcagta tcaactgcgg ct          52


<210>  11
<211>  52
<212>  DNA
<213>  Homo sapiens

<400>  11
agtgccttgt tggatgctga gcccccsata ctctattccg agtatgatcc ta          52


<210>  12
<211>  52
<212>  DNA
<213>  Homo sapiens

<400>  12
agcaggtccc cttcctccta tctcacytat gacgtctcag cctgccttcc ac          52


<210>  13
<211>  51
<212>  DNA

<213>  Homo sapiens

<400>  13
ggagctgggg agcaaacttt ctatamcctg aacactgacc ccacgctcca g          51


<210>  14
<211>  52
<212>  DNA
<213>  Homo sapiens

<400>  14
ttcaaagtcc taacttatcc cagtctygct atccattatt tacttctctc ta          52


<210>  15
<211>  52
<212>  DNA
<213>  Homo sapiens

<400>  15
atatatcctc tgttctttcc actactrcac tatactcctt aatgccaaaa aa          52


<210>  16
<211>  52
<212>  DNA
<213>  Homo sapiens

<400>  16
ccctggggaa gagcagagat atacgtrcca ggtggagcac ccaggcctgg at          52


<210>  17
<211>  52
<212>  DNA
<213>  Homo sapiens

<400>  17
tcatgaacgt gctcaggagg ccatgaygga tgaaacccca gattcttcca aa          52


<210>  18
<211>  52
<212>  DNA
<213>  Homo sapiens

<400>  18
ggcttcagtg tttgtggtta ggaaaasaga aataagaaca acagcagaat gc          52


<210>  19
<211>  52
<212>  DNA
<213>  Homo sapiens

<400>  19
ggaaactgta gactttgctt aaaaagygtc tcatatcatt ttcaaaatag ac          52

<210>  20
<211>  52
<212>  DNA
<213>  Homo sapiens

<400>  20
ttgatgatca tggctaagtg ggacttyagt gactcaaacc ctgtgttcag at          52


<210>  21
<211>  52
<212>  DNA
<213>  Homo sapiens

<400>  21
tagggtgctt aaggacttaa agaacayggg agggaagagg atataacccc aa          52


<210>  22
<211>  52
<212>  DNA
<213>  Homo sapiens

<400>  22
tttggcatta gatgctgttt tgtcttsatg gaaaatacag ctattctagg at          52


<210>  23
<211>  52
<212>  DNA
<213>  Homo sapiens

<400>  23
gaagagtaga gtgggccctt atgtacyttg aagaagtgaa gaatataacc tg          52


<210>  24
<211>  51
<212>  DNA
<213>  Homo sapiens

<400>  24
gctgtgagaa tctgtaaccg atgggkggta actccatggg tgccaacttt g          51


<210>  25
<211>  52
<212>  DNA
<213>  Homo sapiens

<400>  25
gctgccagca gagctccagg ctgaagytgg tgtagtagag gcagcgaacg gt          52


<210>  26
<211>  52
<212>  DNA
<213>  Homo sapiens

<400>  26

gaccagaagg ctctatacac aagagayggc cagctgctgg tgggagatcc tg 52

<210> 27
<211> 52
<212> DNA
<213> Homo sapiens

<400> 27
agccagtagt tcaaggcatt gaacacwgct ccaagcacac cgcctgcgaa ca 52

<210> 28
<211> 52
<212> DNA
<213> Homo sapiens

<400> 28
gccaacaacg gtatgaataa caatatrtcc ttacaagatg ctgaatggta ct 52

<210> 29
<211> 52
<212> DNA
<213> Homo sapiens

<400> 29
agggatgaga ggtgtgtggt ttgcagrttg ggaaacggaa atcacatttg ca 52

<210> 30
<211> 52
<212> DNA
<213> Homo sapiens

<400> 30
ggccagatag ctgttcctga gttgcargca cgatggagat ttggacactg tg 52

<210> 31
<211> 52
<212> DNA
<213> Homo sapiens

<400> 31
attttgctaa ataagtatat attaaayaaa ggtccataac caccatacac at 52

<210> 32
<211> 52
<212> DNA
<213> Homo sapiens

<400> 32
cacacatctg ctctccagga agatgarttt aatgataatg gaatggatgg tg 52

<210> 33
<211> 52
<212> DNA

<213>  Homo sapiens

<400>  33
ggaaggttga acacggtttt ccctgasagt aggctggggt ggggaagtat tg          52


<210>  34
<211>  52
<212>  DNA
<213>  Homo sapiens

<400>  34
aggactgatt cgttatactg agggacrgca gaactagttt cctatgaggg ca          52


<210>  35
<211>  52
<212>  DNA
<213>  Homo sapiens

<400>  35
gatcaaactc acaagtaaca ccaaacyagg aagatcttta ttatctctga ta          52


<210>  36
<211>  52
<212>  DNA
<213>  Homo sapiens

<400>  36
gggattgagc ttcagtcctt tttctamcat cttgttctct aacagaattc tc          52


<210>  37
<211>  52
<212>  DNA
<213>  Homo sapiens

<400>  37
aacaagcatt attcactgag agccttstat ttttcaggca tagtgccaac ag          52


<210>  38
<211>  52
<212>  DNA
<213>  Homo sapiens

<400>  38
cacttcgcca atgccttggt tcgggckctt cctgagactc tcattttcct aa          52


<210>  39
<211>  52
<212>  DNA
<213>  Homo sapiens

<400>  39
gcatccagtg cataatctgc ttgattyatc ttgtctggca cagcataaac ag          52

<210>  40
<211>  52
<212>  DNA
<213>  Homo sapiens

<400>  40
gaaacttgct ttttgagaga ctaagcmaag catctactca cctatgaccc ag          52


<210>  41
<211>  52
<212>  DNA
<213>  Homo sapiens

<400>  41
cagagctcgc tttaccccgg ggaatgmagg tcggggcagt agggaagccc ct          52


<210>  42
<211>  52
<212>  DNA
<213>  Homo sapiens

<400>  42
agacttagga caagccatgg atgcccragc ctgctgactc cactcttgca cc          52


<210>  43
<211>  52
<212>  DNA
<213>  Homo sapiens

<400>  43
tgattctttc tgtgactcat cagttcmttt cctgtaaaat tcatgtcttg ct          52


<210>  44
<211>  52
<212>  DNA
<213>  Homo sapiens

<400>  44
ataatcagaa tcccccactc attggaytgc tgtaaagatt aagagtctca gg          52


<210>  45
<211>  52
<212>  DNA
<213>  Homo sapiens

<400>  45
agcctcctac ttctgctttt gaaagcyata aaaacagcga gggagaaact gg          52


<210>  46
<211>  52
<212>  DNA
<213>  Homo sapiens

<400>  46

aaaacctttg cctcctggag ctaacaytct ggtaccggat agcagatgtg aa 52

<210> 47
<211> 52
<212> DNA
<213> Homo sapiens

<400> 47
agcattcccg ggcgggtgac ccagcayggt ccctcttgga attggattcg cc 52

<210> 48
<211> 52
<212> DNA
<213> Homo sapiens

<400> 48
acatcatggg acagagaaat ccgatgyggt ttgcagttct tctctgccag ct 52

<210> 49
<211> 52
<212> DNA
<213> Homo sapiens

<400> 49
ctctggccca gcaccatctg tgtgatktcc ttccattctt tacccttctt gc 52

<210> 50
<211> 52
<212> DNA
<213> Homo sapiens

<400> 50
ctcttgcaca tcactacctg cagtttygtg gctccctgga acagcctgag ct 52